# EUROPEAN PATENT APPLICATION

(11) **EP 3 865 508 A1**
(43) Date of publication of application: **18.08.2021**
(21) Application number: 19898854.5
(22) Date of filing: 01.10.2019
(51) Int. Cl.: C07K 16/14, C07K 16/40, C12N 9/00, C12N 15/13, C12N 15/63, C12Q 1/686, A61K 38/43, A61K 39/395

(54) **ANTIBODY WHICH FIGHTS TAQ DNA POLYMERASE AND APPLICATION THEREOF**

(30) Priority: 20.12.2018 CN 201811566184
(71) Applicant: Fapon Biotech Inc., Shenzhen City , Guangdong 518000 (CN)
(72) Inventor: CUI, Peng, High-Tech Industrial Development Zone Dongguan, Guangdong 523000 (CN); HE, Zhiqiang, High-Tech Industrial Development Zone Dongguan, Guangdong 523000 (CN); MENG, Yuan, High-Tech Industrial Development Zone Dongguan, Guangdong 523000 (CN); ZHONG, Dongmei, High-Tech Industrial Development Zone Dongguan, Guangdong 523000 (CN); YANG, Hao, High-Tech Industrial Development Zone Dongguan, Guangdong 523000 (CN); LIANG, Bi, High-Tech Industrial Development Zone Dongguan, Guangdong 523000 (CN); YOU, Hui, High-Tech Industrial Development Zone Dongguan, Guangdong 523000 (CN); MA, Qiuyan, High-Tech Industrial Development Zone Dongguan, Guangdong 523000 (CN); LI, Weizhi, High-Tech Industrial Development Zone Dongguan, Guangdong 523000 (CN)
(74) Representative: Biggi, Cristina
(86) International application number: PCT/CN2019/109791
(87) International publication number: WO 2020/125135

(57) **Abstract**

Provided is an isolated binding protein including a Taq DNA polymerase antigen-binding domain. The antigen-binding domain includes at least one complementarity determining region selected from CDR-VH1-3 and CDR-VL1-3, or has at least 80% sequence identity with the complementarity determining region and has an affinity of K_{D}≤8.568×10⁻⁹mol/L to the Taq DNA polymerase. The binding protein may be used in the field of molecular detection.

SEQUENCE LISTING

<110>  FAPON BIOTECH INC.

<120>  ANTI-TAQ DNA POLYMERASE ANTIBODY AND USE THEREOF

<130>  PI19035799

<150>  CN2018115661842
<151>  2018-12-20

<160>  12

<170>  PatentIn version 3.5

<210>  1
<211>  23
<212>  PRT
<213>  Mus musculus

<400>  1

Asp Ile Gln Met Thr Gln Thr Thr Ser Ser Leu Ser Ala Ser Leu Gly
1               5                   10                  15

Asp Arg Val Thr Ile Ser Cys
            20

<210>  2
<211>  13
<212>  PRT
<213>  Mus musculus

<400>  2

Trp Tyr Gln Gln Lys Pro Asp Gly Thr Ile Lys Leu Leu
1               5                   10

<210>  3
<211>  30
<212>  PRT
<213>  Mus musculus

<400>  3

Ser Arg Phe Ser Gly Ser Gly Ser Gly Thr Asp Tyr Ser Leu Thr Ile
1               5                   10                  15

Ala Asn Leu Glu Gln Glu Asp Ile Ala Thr Tyr Phe Cys Gln
            20                  25                  30

<210>  4
<211>  8
<212>  PRT
<213>  Mus musculus

<400>  4

Ala Gly Thr Lys Val Glu Leu Lys
1               5

<210>  5
<211>  24
<212>  PRT
<213>  Mus musculus

<400>  5

Gln Val Gln Leu Gln Gln Pro Gly Ala Glu Leu Val Lys Pro Gly Ala
1               5                   10                  15

Ser Val Arg Leu Ser Cys Gln Thr
            20

<210>  6
<211>  14
<212>  PRT
<213>  Mus musculus

<400>  6

Trp Val Lys Gln Arg Pro Gly Glu Gly Leu Glu Trp Ile Gly
1               5                   10

<210>  7
<211>  33
<212>  PRT
<213>  Mus musculus

<400>  7

Phe Lys Ser Lys Ala Thr Leu Thr Val Asp Thr Ser Ser Ser Thr Ala
1               5                   10                  15

Tyr Met Gln Leu Ser Ser Leu Thr Ser Val Asp Ser Ala Val Tyr Tyr
            20                  25                  30

Cys

<210>  8
<211>  11
<212>  PRT
<213>  Mus musculus

<400>  8

Trp Gly Gln Gly Thr Ala Leu Thr Val Ser Ser
1               5                   10

<210>  9
<211>  107
<212>  PRT

<213>  Mus musculus

<400>  9

Arg Ala Asp Ala Ala Pro Thr Val Ser Ile Phe Pro Pro Ser Ser Glu
1               5                   10                  15

Gln Leu Thr Ser Gly Gly Ala Ser Val Val Cys Phe Leu Asn Asn Phe
            20                  25                  30

Tyr Pro Lys Asp Ile Asn Val Lys Trp Lys Ile Asp Gly Ser Glu Arg
        35                  40                  45

Gln Asn Gly Val Leu Asn Ser Trp Thr Asp Gln Asp Ser Lys Asp Ser
    50                  55                  60

Thr Tyr Ser Met Ser Ser Thr Leu Thr Leu Thr Lys Asp Glu Tyr Glu
65                  70                  75                  80

Arg His Asn Ser Tyr Thr Cys Glu Ala Thr His Lys Thr Ser Thr Ser
                85                  90                  95

Pro Ile Val Lys Ser Phe Asn Arg Asn Glu Cys
            100                 105


<210>  10
<211>  324
<212>  PRT
<213>  Mus musculus

<400>  10

Ala Lys Thr Thr Pro Pro Ser Val Tyr Pro Leu Ala Pro Gly Ser Ala
1               5                   10                  15

Ala Gln Thr Asn Ser Met Val Thr Leu Gly Cys Leu Val Lys Gly Tyr
            20                  25                  30

Phe Pro Glu Pro Val Thr Val Thr Trp Asn Ser Gly Ser Leu Ser Ser
        35                  40                  45

Gly Val His Thr Phe Pro Ala Val Leu Gln Ser Asp Leu Tyr Thr Leu
    50                  55                  60

Ser Ser Ser Val Thr Val Pro Ser Ser Thr Trp Pro Ser Glu Thr Val
65                  70                  75                  80

Thr Cys Asn Val Ala His Pro Ala Ser Ser Thr Lys Val Asp Lys Lys
                85                  90                  95

Ile Val Pro Arg Asp Cys Gly Cys Lys Pro Cys Ile Cys Thr Val Pro
            100                 105                 110

Glu Val Ser Ser Val Phe Ile Phe Pro Pro Lys Pro Lys Asp Val Leu
        115                 120                 125

Thr Ile Thr Leu Thr Pro Lys Val Thr Cys Val Val Val Asp Ile Ser
    130                 135                 140

Lys Asp Asp Pro Glu Val Gln Phe Ser Trp Phe Val Asp Asp Val Glu
145                 150                 155                 160

Val His Thr Ala Gln Thr Gln Pro Arg Glu Glu Gln Phe Asn Ser Thr
                165                 170                 175

Phe Arg Ser Val Ser Glu Leu Pro Ile Met His Gln Asp Trp Leu Asn
            180                 185                 190

Gly Lys Glu Phe Lys Cys Arg Val Asn Ser Ala Ala Phe Pro Ala Pro
        195                 200                 205

Ile Glu Lys Thr Ile Ser Lys Thr Lys Gly Arg Pro Lys Ala Pro Gln
    210                 215                 220

Val Tyr Thr Ile Pro Pro Pro Lys Glu Gln Met Ala Lys Asp Lys Val
225                 230                 235                 240

Ser Leu Thr Cys Met Ile Thr Asp Phe Phe Pro Glu Asp Ile Thr Val
                245                 250                 255

Glu Trp Gln Trp Asn Gly Gln Pro Ala Glu Asn Tyr Lys Asn Thr Gln
            260                 265                 270

Pro Ile Met Asp Thr Asp Gly Ser Tyr Phe Val Tyr Ser Lys Leu Asn
        275                 280                 285

Val Gln Lys Ser Asn Trp Glu Ala Gly Asn Thr Phe Thr Cys Ser Val
    290                 295                 300

Leu His Glu Gly Leu His Asn His His Thr Glu Lys Ser Leu Ser His
305                 310                 315                 320

Ser Pro Gly Lys

<210> 11
<211> 443
<212> PRT

<213>  Mus musculus

<400>  11

Gln Val Gln Leu Gln Gln Pro Gly Ala Glu Leu Val Lys Pro Gly Ala
1               5                   10                  15

Ser Val Arg Leu Ser Cys Gln Thr Ser Val Asp Thr Phe Ser Thr Tyr
            20                  25                  30

Tyr Leu Tyr Trp Val Lys Gln Arg Pro Gly Glu Gly Leu Glu Trp Ile
        35                  40                  45

Gly Gly Val Asn Pro Thr Ser Asn Pro Val Phe Asp Glu Lys Phe Lys
    50                  55                  60

Ser Lys Ala Thr Leu Thr Val Asp Thr Ser Ser Ser Thr Ala Tyr Met
65                  70                  75                  80

Gln Leu Ser Ser Leu Thr Ser Val Asp Ser Ala Val Tyr Tyr Cys Thr
                85                  90                  95

Arg Ser Ile Leu Arg Arg Gly Tyr Tyr Pro Asp Tyr Trp Gly Gln Gly
            100                 105                 110

Thr Ala Leu Thr Val Ser Ser Ala Lys Thr Thr Pro Pro Ser Val Tyr
        115                 120                 125

Pro Leu Ala Pro Gly Ser Ala Ala Gln Thr Asn Ser Met Val Thr Leu
    130                 135                 140

Gly Cys Leu Val Lys Gly Tyr Phe Pro Glu Pro Val Thr Val Thr Trp
145                 150                 155                 160

Asn Ser Gly Ser Leu Ser Ser Gly Val His Thr Phe Pro Ala Val Leu
                165                 170                 175

Gln Ser Asp Leu Tyr Thr Leu Ser Ser Ser Val Thr Val Pro Ser Ser
            180                 185                 190

Thr Trp Pro Ser Glu Thr Val Thr Cys Asn Val Ala His Pro Ala Ser
        195                 200                 205

Ser Thr Lys Val Asp Lys Lys Ile Val Pro Arg Asp Cys Gly Cys Lys
    210                 215                 220

Pro Cys Ile Cys Thr Val Pro Glu Val Ser Ser Val Phe Ile Phe Pro
225                 230                 235                 240

Pro Lys Pro Lys Asp Val Leu Thr Ile Thr Leu Thr Pro Lys Val Thr
                245                 250                 255

Cys Val Val Val Asp Ile Ser Lys Asp Asp Pro Glu Val Gln Phe Ser
            260                 265                 270

Trp Phe Val Asp Asp Val Glu Val His Thr Ala Gln Thr Gln Pro Arg
        275                 280                 285

Glu Glu Gln Phe Asn Ser Thr Phe Arg Ser Val Ser Glu Leu Pro Ile
    290                 295                 300

Met His Gln Asp Trp Leu Asn Gly Lys Glu Phe Lys Cys Arg Val Asn
305                 310                 315                 320

Ser Ala Ala Phe Pro Ala Pro Ile Glu Lys Thr Ile Ser Lys Thr Lys
                325                 330                 335

Gly Arg Pro Lys Ala Pro Gln Val Tyr Thr Ile Pro Pro Pro Lys Glu
            340                 345                 350

Gln Met Ala Lys Asp Lys Val Ser Leu Thr Cys Met Ile Thr Asp Phe
        355                 360                 365

Phe Pro Glu Asp Ile Thr Val Glu Trp Gln Trp Asn Gly Gln Pro Ala
    370                 375                 380

Glu Asn Tyr Lys Asn Thr Gln Pro Ile Met Asp Thr Asp Gly Ser Tyr
385                 390                 395                 400

Phe Val Tyr Ser Lys Leu Asn Val Gln Lys Ser Asn Trp Glu Ala Gly
                405                 410                 415

Asn Thr Phe Thr Cys Ser Val Leu His Glu Gly Leu His Asn His His
            420                 425                 430

Thr Glu Lys Ser Leu Ser His Ser Pro Gly Lys
        435                 440

<210> 12
<211> 214
<212> PRT
<213> Mus musculus

<400> 12

Asp Ile Gln Met Thr Gln Thr Thr Ser Ser Leu Ser Ala Ser Leu Gly
1               5                   10                  15

Asp Arg Val Thr Ile Ser Cys Arg Gly Ser Gln Asp Ile Gln Asn Tyr
            20                  25                  30

Val Asn Trp Tyr Gln Gln Lys Pro Asp Gly Thr Ile Lys Leu Leu Ile
        35                  40                  45

Tyr Phe Thr Ser Arg Leu Gln Ser Gly Ile Pro Ser Arg Phe Ser Gly
    50                  55                  60

Ser Gly Ser Gly Thr Asp Tyr Ser Leu Thr Ile Ala Asn Leu Glu Gln
65                  70                  75                  80

Glu Asp Ile Ala Thr Tyr Phe Cys Gln Gln Asp Asp Thr Ile Pro Val
                85                  90                  95

Thr Trp Gly Ala Gly Thr Lys Val Glu Leu Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

## Description

### Cross-References to Related Application

This application claims the priority of the Chinese patent application with the application No. 201811566184.2, titled "Anti-Taq DNA Polymerase Antibody and Use Thereof" filed to the China National Intellectual Property Administration on December 20, 2018, the entire content of which is incorporated in this application by reference.

### Technical Field

The present disclosure relates to the field of biotechnology and medical technology, in particular to an anti-Taq DNA polymerase antibody and use thereof.

### Background

In 1988, Randall K. Saiki et al. isolated and purified Taq DNA polymerase that could tolerate the high temperature above 90°C without inactivation from *Thermus aquaticus,* which was significant in PCR reaction that required a high temperature environment. Therefore, Taq polymerase replaced the DNA polymerase in *Escherichia coli* that was previously commonly used in PCR reaction. There is no need to add enzyme in every cycle by using Taq polymerase in PCR reaction, which makes the PCR technology very convenient with much lower cost, thus the PCR technology is widely used and gradually applied in clinical practice. However, Taq DNA polymerase also has some defects in its application, that is, it also has some enzymatic properties at room temperature, which leads to non-specific amplification and primer dimer formation during PCR amplification, as well as long-term stability problems.

Therefore, with the popularization of PCR technology application and the improvement of its quality requirements, new methods and technologies continue to emerge. Among them, the emergence of hot start enzyme technology can qualitatively improve the enzymatic properties of ordinary Taq DNA polymerases. Currently, the commonly used methods include an antibody-modified hot start enzyme that are more commonly used, and a chemically-modified hot start enzyme.

The antibody-modified hot start enzyme is a monoclonal antibody that requires specific Taq enzyme. The monoclonal antibody of specific Taq enzyme binds to Taq DNA polymerase to form an antigen-antibody complex, which can effectively block the activity of Taq DNA polymerase at room temperature, so that it does not exert polymerase activity under low temperature conditions, while this complex will dissociate at high temperature to release active Taq DNA polymerase, which can effectively avoid the formation of primer dimers, reduce the amplification of non-specific products and improve the long-term stability of Taq DNA polymerase for PCR amplification reaction.

The antibody for specific Taq enzyme used in the antibody-modified hot start enzyme needs to be further developed.

### Summary

The present disclosure relates to a novel isolated binding protein including a Taq DNA polymerase antigen-binding domain, and investigates the preparation, use and other aspects of the binding protein.

The antigen-binding domain includes at least one complementarity determining region selected from the following amino acid sequences, or has at least 80% sequence identity with the complementarity determining region of the following amino acid sequences and has an affinity of K_{D}≤8.568×10⁻⁹mol/L to a Taq DNA polymerase;
the complementarity determining region CDR-VH1 is S-V-X1-T-F-X2-T-Y-Y-X3-Y, wherein
X1 is D, E or N, X2 is S or T, X3 is I or L;
the complementarity determining region CDR-VH2 is G-X1-N-P-T-S-X2-P-V-F-X3-E-K, wherein
X1 is I, V or L, X2 is N or GG, X3 is D, E or N;
the complementarity determining region CDR-VH3 is T-R-S-X1-X2-R-R-G-Y-Y -X3-D-Y, wherein
X1 is I, V or L, X2 is I, V or L, X3 is F or P;
the complementarity determining region CDR-VL1 is R-X1-S-Q-D-I-X2-N-N-Y-X3-N, wherein
X1 is A or G, X2 isN orQ, X3 is I, V orL;
the complementarity determining region CDR-VL2 is I-Y-X1-T-S-R-L-X2-S-G-X3-P, wherein
X1 is Y or F, X2 is Q, H or N, X3 is I, V or L;
the complementarity determining region CDR-VL3 is Q-D-D-T-X1-P-X2-T-X3-G, wherein

### Description

X1 is I, V or L, X2 is I, V or L, and X3 is W or F.

The binding protein has an important advantage in that it has strong activity and high affinity to Taq DNA polymerase.

In one or more embodiments,
X3 is I in the complementarity determining region CDR-VH1;
X3 is N in the complementarity determining region CDR-VH2;
X3 is F in the complementarity determining region CDR-VH3;
X1 is A in the complementarity determining region CDR-VL1;
X1 is Y in the complementarity determining region CDR-VL2;
X3 is F in the complementarity determining region CDR-VL3.

In one or more embodiments, X1 is D and X2 is S in the complementarity determining region CDR-VH1.

In one or more embodiments, X1 is E and X2 is S in the complementarity determining region CDR-VH1.

In one or more embodiments, X1 is N and X2 is S in the complementarity determining region CDR-VH1.

In one or more embodiments, X1 is D and X2 is T in the complementarity determining region CDR-VH1.

In one or more embodiments, X1 is E and X2 is T in the complementarity determining region CDR-VH1.

In one or more embodiments, X1 is N and X2 is T in the complementarity determining region CDR-VH1.

In one or more embodiments, X1 is I and X2 is N in the complementarity determining region CDR-VH2.

In one or more embodiments, X1 is I and X2 is GG in the complementarity determining region CDR-VH2.

In one or more embodiments, X1 is V and X2 is N in the complementarity determining region CDR-VH2.

In one or more embodiments, X1 is V and X2 is GG in the complementarity determining region CDR-VH2.

In one or more embodiments, X1 is L and X2 is N in the complementarity determining region CDR-VH2.

In one or more embodiments, X1 is L and X2 is GG in the complementarity determining region CDR-VH2.

In one or more embodiments, X1 is I and X2 is I in the complementarity determining region CDR-VH3.

In one or more embodiments, X1 is I and X2 is V in the complementarity determining region CDR-VH3.

In one or more embodiments, X1 is I and X2 is L in the complementarity determining region CDR-VH3.

In one or more embodiments, X1 is V and X2 is I in the complementarity determining region CDR-VH3.

In one or more embodiments, X1 is V and X2 is V in the complementarity determining region CDR-VH3.

In one or more embodiments, X1 is V and X2 is L in the complementarity determining region CDR-VH3.

In one or more embodiments, X1 is L and X2 is I in the complementarity determining region CDR-VH3.

In one or more embodiments, X1 is L and X2 is V in the complementarity determining region CDR-VH3.

In one or more embodiments, X1 is L and X2 is L in the complementarity determining region CDR-VH3.

In one or more embodiments, X2 is N and X3 is I in the complementarity determining region CDR-VL1.

In one or more embodiments, X2 is N and X3 is V in the complementarity determining region CDR-VL1.

In one or more embodiments, X2 is N and X3 is L in the complementarity determining region CDR-VL1.

In one or more embodiments, X2 is Q and X3 is I in the complementarity determining region CDR-VL1.

In one or more embodiments, X2 is Q and X3 is V in the complementarity determining region CDR-VL1.

In one or more embodiments, X2 is Q and X3 is L in the complementarity determining region CDR-VL1.

In one or more embodiments, X2 is Q and X3 is I in the complementarity determining region CDR-VL2.

In one or more embodiments, X2 is Q and X3 is V in the complementarity determining region CDR-VL2.

In one or more embodiments, X2 is Q and X3 is L in the complementarity determining region CDR-VL2.

In one or more embodiments, X2 is H and X3 is I in the complementarity determining region CDR-VL2.

In one or more embodiments, X2 is H and X3 is V in the complementarity determining region CDR-VL2.

In one or more embodiments, X2 is H and X3 is L in the complementarity determining region CDR-VL2.

In one or more embodiments, X2 is N and X3 is I in the complementarity determining region CDR-VL2.

In one or more embodiments, X2 is N and X3 is V in the complementarity determining region CDR-VL2.

In one or more embodiments, X2 is N and X3 is L in the complementarity determining region CDR-VL2.

In one or more embodiments, X1 is I and X2 is I in the complementarity determining region CDR-VL3.

In one or more embodiments, X1 is I and X2 is V in the complementarity determining region CDR-VL3.

In one or more embodiments, X1 is I and X2 is L in the complementarity determining region CDR-VL3.

In one or more embodiments, X1 is V and X2 is I in the complementarity determining region CDR-VL3.

In one or more embodiments, X1 is V and X2 is V in the complementarity determining region CDR-VL3.

In one or more embodiments, X1 is V and X2 is L in the complementarity determining region CDR-VL3.

In one or more embodiments, X1 is L and X2 is I in the complementarity determining region CDR-VL3.

In one or more embodiments, X1 is L and X2 is V in the complementarity determining region CDR-VL3.

In one or more embodiments, X1 is L and X2 is L in the complementarity determining region CDR-VL3.

In one or more embodiments, a mutation site of each complementarity determining region is selected from any one of the following mutation combinations:

| Site | CDR-VH1 X1/X2 | CDR-VH2 X1/X2 | CDR-VH3 X1/X2 | CDR-VL1 X2/X3 | CDR-VL2 X2/X3 | CDR-VL3 X1/X2 |
|---|---|---|---|---|---|---|
| Mutation combination 1 | D/S | V/N | I/L | Q/V | Q/I | I/V |
| Mutation combination 2 | D/T | I/N | I/V | N/V | Q/V | I/I |
| Mutation combination 3 | E/S | L/N | I/I | Q/L | Q/L | I/L |
| Mutation combination 4 | E/T | V/GG | V/L | N/L | H/I | V/V |
| Mutation combination 5 | N/S | I/GG | V/V | Q/I | H/V | V/I |
| Mutation combination 6 | N/T | L/GG | V/I | N/I | H/L | V/L |
| Mutation combination 7 | D/S | V/N | L/L | Q/V | N/I | L/V |
| Mutation combination 8 | D/T | I/N | L/V | N/V | N/V | L/I |
| Mutation combination 9 | E/S | L/N | L/I | Q/L | N/L | L/L |
| Mutation combination 10 | E/T | V/GG | I/L | N/L | Q/I | I/V |
| Mutation combination 11 | N/S | I/GG | I/V | Q/I | Q/V | I/I |
| Mutation combination 12 | N/T | L/GG | I/I | N/I | Q/L | I/L |
| Mutation combination 13 | D/S | V/N | V/L | Q/V | H/I | V/V |
| Mutation combination 14 | D/T | I/N | V/V | N/V | H/V | V/I |
| Mutation combination 15 | E/S | L/N | V/I | Q/L | H/L | V/L |
| Mutation combination 16 | E/T | V/GG | L/L | N/L | N/I | L/V |
| Mutation combination 17 | N/S | I/GG | L/V | Q/I | N/V | L/I |
| Mutation combination 18 | N/T | L/GG | L/I | N/I | N/L | L/L |
| Mutation combination 19 | D/T | V/N | I/L | Q/V | Q/I | I/V |
| Mutation combination 20 | E/S | I/N | I/V | N/V | H/I | I/I |
| Mutation combination 21 | E/T | L/N | I/I | Q/L | N/I | I/L |
| Mutation combination 22 | N/S | V/GG | V/L | N/L | Q/V | V/V |
| Mutation combination 23 | N/T | I/GG | V/V | Q/I | H/V | V/I |
| Mutation combination 24 | D/S | L/GG | V/I | N/I | N/V | V/L |
| Mutation combination 25 | E/S | V/GG | L/L | Q/V | Q/L | L/V |
| Mutation combination 26 | E/T | I/GG | L/V | N/V | H/L | L/I |
| Mutation combination 27 | N/S | L/GG | L/I | Q/L | N/L | L/L |
| Mutation combination 28 | N/T | V/N | I/L | N/L | Q/I | I/V |
| Mutation combination 29 | D/S | I/N | I/V | Q/I | H/I | I/I |
| Mutation combination 30 | E/T | L/N | I/I | N/I | N/I | I/V |
| Mutation combination 31 | N/S | I/GG | V/L | Q/V | Q/V | VL |
| Mutation combination 32 | N/T | L/GG | V/V | N/V | H/V | V/I |
| Mutation combination 33 | D/S | V/GG | V/I | Q/L | N/V | V/V |
| Mutation combination 34 | E/S | I/GG | L/L | N/L | Q/L | L/L |
| Mutation combination 35 | D/S | L/GG | L/V | Q/I | H/L | L/I |
| Mutation combination 36 | D/T | V/N | L/I | N/I | N/L | L/V |
| Mutation combination 37 | E/S | I/N | I/L | Q/V | Q/I | I/L |
| Mutation combination 38 | E/T | L/N | I/V | N/V | H/I | I/I |
| Mutation combination 39 | N/S | V/GG | I/I | Q/L | N/I | I/L |
| Mutation combination 40 | N/T | I/GG | V/L | N/L | Q/V | V/L |
| Mutation combination 41 | D/S | L/GG | V/V | Q/I | H/V | V/I |
| Mutation combination 42 | D/T | V/N | V/I | N/I | N/V | V/V |
| Mutation combination 43 | E/S | I/N | L/L | Q/V | Q/L | L/L |
| Mutation combination 44 | E/T | L/N | L/V | N/V | H/L | L/I |
| Mutation combination 45 | N/S | V/GG | L/I | Q/L | N/L | L/V |
| Mutation combination 46 | N/T | I/GG | I/L | N/L | N/V | I/L |
| Mutation combination 47 | D/S | L/GG | I/V | Q/I | N/I | I/I |
| Mutation combination 48 | D/T | V/N | I/I | N/I | N/L | I/V |
| Mutation combination 49 | E/S | I/N | V/L | Q/V | Q/V | V/L |
| Mutation combination 50 | E/T | L/N | V/V | N/V | Q/I | V/I |
| Mutation combination 51 | N/S | V/GG | V/I | Q/L | Q/L | V/V |
| Mutation combination 52 | N/T | I/GG | L/L | N/L | H/V | L/L |
| Mutation combination 53 | E/T | L/GG | L/V | Q/I | H/I | L/I |
| Mutation combination 54 | N/S | I/N | L/I | N/I | H/L | L/V |
| Mutation combination 55 | N/T | L/N | I/L | Q/V | N/V | I/V |
| Mutation combination 56 | D/S | V/GG | I/V | N/V | N/I | I/I |
| Mutation combination 57 | D/T | I/GG | I/I | Q/L | N/L | I/L |
| Mutation combination 58 | E/S | V/N | V/L | N/L | Q/V | V/V |
| Mutation combination 59 | E/T | I/N | V/V | Q/I | Q/I | V/I |
| Mutation combination 60 | N/S | L/N | V/I | N/I | Q/L | V/L |
| Mutation combination 61 | N/T | V/GG | L/L | Q/I | H/V | L/V |

In one or more embodiments, the binding protein includes at least 3 CDRs; alternatively, the binding protein includes at least 6 CDRs.

In one or more embodiments, the binding protein is an intact antibody including a variable region and a constant region.

In one or more embodiments, the binding protein is one of a nanobody, F(ab')2, Fab', Fab, Fv, scFv, a bispecific antibody and a minimal recognition unit of antibody.

In one or more embodiments, the binding protein includes light chain framework regions FR-L1, FR-L2, FR -L3 and FR-L4 with sequence correspondingly shown in SEQ ID NO: 1-4, and/or, heavy chain framework regions FR-H1, FR-H2, FR-H3 and FR-H4 with sequence correspondingly shown in SEQ ID NO: 5-8.

In one or more embodiments, the binding protein further includes an antibody constant region sequence.

In one or more embodiments, the constant region sequence is a sequence of any one constant region selected from IgG1, IgG2, IgG3, IgG4, IgA, IgM, IgE, and IgD.

In one or more embodiments, the constant region is derived from species consisted of cattle, horse, dairy cow, pig, sheep, goat, rat, mouse, dog, cat, rabbit, camel, donkey, deer, mink, chicken, duck, goose, turkey, gamecock or human.

In one or more embodiments, the constant region is derived from the mouse;
a light chain constant region sequence is shown in SEQ ID NO: 9;
a heavy chain constant region sequence is shown in SEQ ID NO: 10.

The present disclosure also provides an isolated nucleic acid, encoding the above binding protein.

The present disclosure also provides a vector, including the above nucleic acid.

The expression vector of the present disclosure is used to transform a host cell.

Such transformed cell is also as part of the present disclosure, and may be a cultured cell or cell strain used to propagate the nucleic acid fragment and vector of the present disclosure, or to recombinantly prepare the polypeptide of the present disclosure.

The present disclosure also provides a method for producing the above binding protein, including the steps of:
culturing the above host cell in a culture medium, recovering a produced binding protein from the culture medium or from the cultured host cell.

The present disclosure also provides a use of the above binding protein in PCR.

The present disclosure also provides a kit, including one or more of the above binding proteins, the above isolated nucleic acid, or the above vector.

The present disclosure also provides a composition, including the binding protein of the present disclosure and a Taq DNA polymerase.

In one or more embodiments, the composition further includes 4 deoxynucleoside triphosphates.

In one or more embodiments, the composition further includes a primer and/or a probe.

In one or more embodiments, the composition further includes MgCl₂.

In one or more embodiments, the composition further includes a nucleic acid as a template.

The present disclosure also provides a method for amplifying nucleic acid, including carrying out hot start PCR by using the binding protein of the present disclosure or the composition of the present disclosure.

In one or more embodiments, the hot start PCR is selected from a group consisted of multiplex PCR, real-time PCR, and real-time quantitative PCR.

The present disclosure also provides a method for detecting Taq DNA polymerase in a test sample, including the steps of:
a) contacting the Taq DNA polymerase in the test sample with the binding protein of the present disclosure to form an immune complex under conditions sufficient for an antibody/antigen binding reaction to occur; and
b) detecting the presence of the immune complex, which indicates the presence of the Taq DNA polymerase in the test sample.

In one or more embodiments, the immune complex further includes a second antibody that binds to the binding protein;

In one or more embodiments, in step a), the immune complex further includes a second antibody that binds to the Taq DNA polymerase.

The present disclosure also provides a use of the binding protein described herein in amplifying a nucleic acid.

### Brief Description of the Drawings

In order to more clearly illustrate the specific embodiments of the present disclosure or the technical solutions in the prior art, specific embodiments or drawings that may be required in prior art descriptions are briefly described below, obviously, the drawings described below are some embodiments of the present disclosure. For those of ordinary skill in the art, other drawings can be obtained based on these drawings without creative work.
FIG. 1 is an electrophoretogram of a monoclonal antibody of the anti-Taq DNA polymerase recombinant antibody of the disclosure.
FIG. 2 shows the PCR electrophoretogram of Taq DNA polymerase.

### Detailed Description of the Embodiments

The present disclosure can be more easily understood through the following description of some embodiments of the present disclosure and the detailed content of the embodiments included therein.

Before further describing the present disclosure, it should be understood that the present disclosure is not limited to the specific embodiments, because these embodiments are necessarily diverse. It should also be understood that the terms used in this specification are only to illustrate specific embodiments, rather than as limitations, because the scope of the present disclosure will be defined in the appended claims.

Unless otherwise defined herein, scientific and technical terms used together with this disclosure shall have the meanings commonly understood by those of ordinary skill in the art. The meaning and scope of the terms should be clear, however, in any case of potential ambiguity, the definitions provided herein take precedence over any dictionary or foreign definitions. In this disclosure, the use of "or" means "and/or" unless stated otherwise. In addition, the use of the term "including" and other forms is non-limiting.

Generally, the nomenclature and techniques used together with cell and tissue culture, molecular biology, immunology, microbiology, genetics, as well as protein and nucleic acid chemistry and hybridization described herein are those well known and commonly used in the art. Unless otherwise stated, the methods and techniques of the present disclosure are generally performed according to conventional methods well known in the art and as described in various general and more specific references, which are cited and discussed throughout this specification. Enzymatic reactions and purification techniques are performed according to the manufacturer's instruction, as commonly achieved in the art, or as described herein. Together with the nomenclature used in analytical chemistry, synthetic organic chemistry, and medical and pharmaceutical chemistry described herein, as well as their laboratory procedures and techniques are those well known and commonly used in the art.

In order for the present disclosure to be more easily understood, selected terms are defined below.

The term "amino acid" refers to a naturally occurring or a non-naturally occurring carboxy alpha-amino acid. The term "amino acid" as used in this disclosure can include a naturally occurring amino acid and a non-naturally occurring amino acid. The naturally occurring amino acid includes alanine (three letter code: Ala, one letter code: A), arginine (Arg, R), asparagine (Asn, N), aspartic acid (Asp, D), Cysteine (Cys, c), glutamine (Gln, Q), glutamic acid (Glu, E), glycine (Gly, G), histidine (His, H), isoleucine (Ile , I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V). The non-naturally occurring amino acid includes but is not limited to α-aminoadipate, aminobutyric acid, citrulline, homocitrulline, homoleucine, homoarginine, hydroxyproline, norleucine, pyridylalanine, sarcosine and so on.

The term "isolated binding protein" is a such protein that, due to its derived origin or source does not bind to a naturally-associated component, which is accompanied by it in the natural state; is substantially free of other proteins from the same species; is expressed by cells from different species; or is non-existent in nature Therefore, a protein synthesized chemically or synthesized in a cell system different from the cell of its natural origin which will be "isolated" from its naturally bound component. It can also be isolated, for example, using protein purification techniques well known in the art, so that the protein is substantially free of naturally bound components.

The term "isolated binding protein including an antigen binding domain" broadly refers to all proteins/protein fragments including a CDR region. The term "antibody" includes a polyclonal antibody, a monoclonal antibody, and the antigen compound binding fragments of these antibodies, including Fab, F(ab')2, Fd, Fv, scFv, a bispecific antibody and a minimum recognition unit of antibody, as well as single-chain derivatives of these antibodies and fragments. The type of antibody can select from IgG1, IgG2, IgG3, IgG4, IgA, IgM, IgE and IgD. In addition, the term "antibody" includes a naturally-occurring antibody and a non-naturally-occurring antibody, including, for example, chimeric, bifunctional, and humanized antibodies, and related synthetic isoforms. The term "antibody" can be used interchangeably with "immunoglobulin".

A "variable region" or "variable domain" of an antibody refers to a amino terminal domain of a heavy or light chain of an antibody. A variable domain of a heavy chain can be referred to as "VH". A variable domain of a light chain can be referred to as "VL". These domains are usually the most variable part of the antibody and contain the antigen binding site. A variable region of a light chain or a heavy chain is consisted of three called "complementarity determining regions" or "CDRs" and the framework regions that separate the three complementarity determining regions. A framework region of an antibody, that is, a framework region that constitutes a combination of an essential light chain and a heavy chain, plays a role in positioning and aligning the CDR that are mainly responsible for binding to the antigen.

As used herein, "framework region", "framework regions" or "FR" means that the excluded antibody variable domains are regions other than those defined as CDRs. Each antibody variable domain framework region can be further subdivided into adjacent regions (FR1, FR2, FR3, and FR4) separated by CDRs.

Generally, the variable regions VL/VH of the heavy chain and the light chain can be obtained by arranging and linking the following numbered CDRs and FRs in the following combination: FR1-CDR1-FR2-CDR2-FR3-CDR3-FR4.

As used herein, the term "purified" or "isolated" associated with a polypeptide or nucleic acid means that the polypeptide or nucleic acid is not in its natural medium or in its natural form. Thus, the term "isolated" includes a polypeptide or nucleic acid taken from its original environment, for example, from the natural environment if it is naturally occurring. For example, an isolated polypeptide generally does not contain at least some proteins or other cellular components that are normally bound to it or usually mixed with it or in solution. An isolated polypeptide includes a naturally produced polypeptide contained in the cell lysate, a polypeptide in purified or partially purified form, a recombinant polypeptide, a polypeptide expressed or secreted by a cell, and a polypeptide in a heterologous host cell or the culture. Associated with nucleic acid, the term isolated or purified indicates that, for example, the nucleic acid is not in its natural genomic context (for example, in a vector, as an expression cassette, linked to a promoter, or artificially introduced into a heterologous host cell).

As used herein, the term "bispecific antibody" or "bifunctional antibody" refers to an artificial hybrid binding protein with two different pairs of heavy/light chains and two different binding sites. The bispecific binding protein can be produced by a variety of methods, including fusion of hybridomas or linking of Fab' fragments. As used herein, the term "sequence identity" refers to the similarity between at least two different sequences. The percentage identity can be determined by standard algorithms, such as Basic Local Alignment Search Tool (BLAST); an algorithm described by Needleman et al.; or an algorithm described by Meyers et al. In one or more embodiments, a set of parameters may be Blosum 62 scoring matrix and gap penalty of 12, gap extension penalty of 4, and frameshift gap penalty of 5. In one or more embodiments, the percentage identity between two amino acid or nucleotide sequences can also be determined using the algorithm described by Meyers and Miller ((1989) CABIOS 4: 11-17), which has been incorporated to the ALIGN program (version 2.0), using the PAM120 weight residue table, gap length penalty of 12, and gap penalty of 4. Percentage identity is usually calculated by comparing sequences having similar length.

As used herein, the term "affinity" refers to the binding strength of an antigen binding domain of a binding protein or antibody to an antigen or antigen epitope. Affinity can be measured by the KD value, the smaller of which, the greater the affinity.

The present disclosure provides an isolated binding protein including an antigen-binding domain. The antigen-binding domain includes at least one complementarity determining region selected from the following amino acid sequences, or has at least 80% sequence identity with the complementarity determining region of the following amino acid and has an affinity of K_{D}≤8.568×10⁻⁹mol/L to the Taq DNA polymerase;
the complementarity determining region CDR-VH1 is S-V-X1-T-F-X2-T-Y-Y-X3-Y, wherein
X1 is D, E or N, X2 is S or T, X3 is I or L;
the complementarity determining region CDR-VH2 is G-X1-N-P-T-S-X2-P-V-F-X3-E-K, wherein
X1 is I, V or L, X2 is N or GG, X3 is D, E or N;
the complementarity determining region CDR-VH3 is T-R-S-X1-X2-R-R-G-Y-Y-X3-D-Y, wherein
X1 is I, V or L, X2 is I, V or L, X3 is F or P;
the complementarity determining region CDR-VL1 is R-X1-S-Q-D-I-X2-N-Y-X3-N, wherein
X1 is A or G, X2 isN orQ, X3 is I, V orL;
the complementarity determining region CDR-VL2 is I-Y-X1-T-S-R-L-X2-S-G-X3-P, wherein
X1 is Y or F, X2 is Q, H or N, X3 is I, V or L;
the complementarity determining region CDR-VL3 is Q-D-D-T-X1-P-X2-T-X3-G, wherein
X1 is I, V or L, X2 is I, V or L, and X3 is W or F.

It is well known in the art that the binding specificity and affinity of an antibody are mainly determined by the CDR sequence, and the amino acid sequence of the non-CDR region can be easily altered to obtain variants with similar biological activity, according to mature and well-known existing technologies. Therefore, the present disclosure also includes "functional derivative" of the binding protein. "Functional derivative" refers to a variant of amino acid substitution. One functional derivative retains detectable protein binding activity, preferably the activity of an antibody capable of binding Taq DNA polymerase. "Functional derivative" may include "variant" and "fragment", because they have exactly identical CDR sequence as the binding protein described in the present disclosure, and therefore have similar biological activities.

In one or more embodiments, the antigen binding domain has at least 50%, or at least 55%, or at least 60%, or at least 65%, or at least 70%, Or at least 75%, or at least 80%, or at least 85%, or at least 90%, or at least 91%, or at least 92%, or at least 93%, or at least 94%, or at least 95%, or at least 96%, or at least 97%, or at least 98%, or at least 99% sequence identity with the complementarity determining region of the following amino acid sequence and having an affinity of K_{D}≤8.568×10⁻⁹mol/L to Taq DNA polymerase. For example, 8.568×10⁻⁹mol/L, 5.126×10⁻⁹mol/L, 3.018×10⁻⁹mol/L, 2.196×10⁻¹⁰mol/L, 3.839×10⁻¹⁰mol/L, 4.075×10⁻¹⁰mol/L, 6.772×10⁻¹⁰mol/L, 8.499×10⁻¹⁰mol/L, 9.870×10⁻¹⁰mol/L, 3.145×10⁻¹¹mol/L, 5.067×10⁻¹¹mol/L, 6.643×10⁻¹¹mol/L, or 1.328×10⁻¹¹mol/L K_{D}≤8.568×10⁻⁹mol/L, or 1.328×10⁻¹¹mol/L≤KD≤9.870×10⁻¹⁰mol/L; or KD less or equal to 5.126×10⁻⁹mol/L; 3.018×10⁻⁹mol/L, 2.196×10⁻¹⁰mol/L, 3.839×10⁻¹⁰mol/L, 4.075×10⁻¹⁰mol/L, 6.772×10⁻¹⁰mol/L, 8.499×10⁻¹⁰mol/L, 9.870×10⁻¹⁰mol/L, 3.145×10⁻¹¹mol/L, 5.067×10⁻¹¹mol/L or 6.643×10⁻¹¹mol/L.

Wherein, the affinity is measured according to the method in the present disclosure.

In one or more embodiments,
X3 is I in the complementarity determining region CDR-VH1;
X3 is N in the complementarity determining region CDR-VH2;
X3 is F in the complementarity determining region CDR-VH3;
X1 is A in the complementarity determining region CDR-VL1;
X1 is Y in the complementarity determining region CDR-VL2;
X3 is F in the complementarity determining region CDR-VL3.

In one or more embodiments, X1 is D and X2 is S in the complementarity determining region CDR-VH1.

In one or more embodiments, X1 is E and X2 is S in the complementarity determining region CDR-VH1.

In one or more embodiments, X1 is N and X2 is S in the complementarity determining region CDR-VH1.

In one or more embodiments, X1 is D and X2 is T in the complementarity determining region CDR-VH1.

In one or more embodiments, X1 is E and X2 is T in the complementarity determining region CDR-VH1.

In one or more embodiments, X1 is N and X2 is T in the complementarity determining region CDR-VH1.

In one or more embodiments, X1 is I and X2 is N in the complementarity determining region CDR-VH2.

In one or more embodiments, X1 is I and X2 is GG in the complementarity determining region CDR-VH2.

In one or more embodiments, X1 is V and X2 is N in the complementarity determining region CDR-VH2.

In one or more embodiments, X1 is V and X2 is GG in the complementarity determining region CDR-VH2.

In one or more embodiments, X1 is L and X2 is N in the complementarity determining region CDR-VH2.

In one or more embodiments, X1 is L and X2 is GG in the complementarity determining region CDR-VH2.

In one or more embodiments, X1 is I and X2 is I in the complementarity determining region CDR-VH3.

In one or more embodiments, X1 is I and X2 is V in the complementarity determining region CDR-VH3.

In one or more embodiments, X1 is I and X2 is L in the complementarity determining region CDR-VH3.

In one or more embodiments, X1 is V and X2 is I in the complementarity determining region CDR-VH3.

In one or more embodiments, X1 is V and X2 is V in the complementarity determining region CDR-VH3.

In one or more embodiments, X1 is V and X2 is L in the complementarity determining region CDR-VH3.

In one or more embodiments, X1 is L and X2 is I in the complementarity determining region CDR-VH3.

In one or more embodiments, X1 is L and X2 is V in the complementarity determining region CDR-VH3.

In one or more embodiments, X1 is L and X2 is L in the complementarity determining region CDR-VH3.

In one or more embodiments, X2 is N and X3 is I in the complementarity determining region CDR-VL1.

In one or more embodiments, X2 is N and X3 is V in the complementarity determining region CDR-VL1.

In one or more embodiments, X2 is N and X3 is L in the complementarity determining region CDR-VL1.

In one or more embodiments, X2 is Q and X3 is I in the complementarity determining region CDR-VL1.

In one or more embodiments, X2 is Q and X3 is V in the complementarity determining region CDR-VL1.

In one or more embodiments, X2 is Q and X3 is L in the complementarity determining region CDR-VL1.

In one or more embodiments, X2 is Q and X3 is I in the complementarity determining region CDR-VL2.

In one or more embodiments, X2 is Q and X3 is V in the complementarity determining region CDR-VL2.

In one or more embodiments, X2 is Q and X3 is L in the complementarity determining region CDR-VL2.

In one or more embodiments, X2 is H and X3 is I in the complementarity determining region CDR-VL2.

In one or more embodiments, X2 is H and X3 is V in the complementarity determining region CDR-VL2.

In one or more embodiments, X2 is H and X3 is L in the complementarity determining region CDR-VL2.

In one or more embodiments, X2 is N and X3 is I in the complementarity determining region CDR-VL2.

In one or more embodiments, X2 is N and X3 is V in the complementarity determining region CDR-VL2.

In one or more embodiments, X2 is N and X3 is L in the complementarity determining region CDR-VL2.

In one or more embodiments, X1 is I and X2 is I in the complementarity determining region CDR-VL3.

In one or more embodiments, X1 is I and X2 is V in the complementarity determining region CDR-VL3.

In one or more embodiments, X1 is I and X2 is L in the complementarity determining region CDR-VL3.

In one or more embodiments, X1 is V and X2 is I in the complementarity determining region CDR-VL3.

In one or more embodiments, X1 is V and X2 is V in the complementarity determining region CDR-VL3.

In one or more embodiments, X1 is V and X2 is L in the complementarity determining region CDR-VL3.

In one or more embodiments, X1 is L and X2 is I in the complementarity determining region CDR-VL3.

In one or more embodiments, X1 is L and X2 is V in the complementarity determining region CDR-VL3.

In one or more embodiments, X1 is L and X2 is L in the complementarity determining region CDR-VL3.

In one or more embodiments, a mutation site of each complementarity determining region is selected from any one of the following mutation combinations:

| Site | CDR-VH1 X1/X2 | CDR-VH2 X1/X2 | CDR-VH3 X1/X2 | CDR-VL1 X2/X3 | CDR-VL2 X2/X3 | CDR-VL3 X1/X2 |
|---|---|---|---|---|---|---|
| Mutation combination 1 | D/S | V/N | I/L | Q/V | Q/I | I/V |
| Mutation combination 2 | D/T | I/N | I/V | N/V | Q/V | I/I |
| Mutation combination 3 | E/S | L/N | I/I | Q/L | Q/L | I/L |
| Mutation combination 4 | E/T | V/GG | V/L | N/L | H/I | V/V |
| Mutation combination 5 | N/S | I/GG | V/V | Q/I | H/V | V/I |
| Mutation combination 6 | N/T | L/GG | V/I | N/I | H/L | V/L |
| Mutation combination 7 | D/S | V/N | L/L | Q/V | N/I | L/V |
| Mutation combination 8 | D/T | I/N | L/V | N/V | N/V | L/I |
| Mutation combination 9 | E/S | L/N | L/I | Q/L | N/L | L/L |
| Mutation combination 10 | E/T | V/GG | I/L | N/L | Q/I | I/V |
| Mutation combination 11 | N/S | I/GG | I/V | Q/I | Q/V | I/I |
| Mutation combination 12 | N/T | L/GG | I/I | N/I | Q/L | I/L |
| Mutation combination 13 | D/S | V/N | V/L | Q/V | H/I | V/V |
| Mutation combination 14 | D/T | I/N | V/V | N/V | H/V | V/I |
| Mutation combination 15 | E/S | L/N | V/I | Q/L | H/L | V/L |
| Mutation combination 16 | E/T | V/GG | L/L | N/L | N/I | L/V |
| Mutation combination 17 | N/S | I/GG | L/V | Q/I | N/V | L/I |
| Mutation combination 18 | N/T | L/GG | L/I | N/I | N/L | L/L |
| Mutation combination 19 | D/T | V/N | I/L | Q/V | Q/I | I/V |
| Mutation combination 20 | E/S | I/N | I/V | N/V | H/I | I/I |
| Mutation combination 21 | E/T | L/N | I/I | Q/L | N/I | I/L |
| Mutation combination 22 | N/S | V/GG | V/L | N/L | Q/V | V/V |
| Mutation combination 23 | N/T | I/GG | V/V | Q/I | H/V | V/I |
| Mutation combination 24 | D/S | L/GG | V/I | N/I | N/V | V/L |
| Mutation combination 25 | E/S | V/GG | L/L | Q/V | Q/L | L/V |
| Mutation combination 26 | E/T | I/GG | L/V | N/V | H/L | L/I |
| Mutation combination 27 | N/S | L/GG | L/I | Q/L | N/L | L/L |
| Mutation combination 28 | N/T | V/N | I/L | N/L | Q/I | I/V |
| Mutation combination 29 | D/S | I/N | I/V | Q/I | H/I | I/I |
| Mutation combination 30 | E/T | L/N | I/I | N/I | N/I | I/V |
| Mutation combination 31 | N/S | I/GG | V/L | Q/V | Q/V | VL |
| Mutation combination 32 | N/T | L/GG | V/V | N/V | H/V | V/I |
| Mutation combination 33 | D/S | V/GG | V/I | Q/L | N/V | V/V |
| Mutation combination 34 | E/S | I/GG | L/L | N/L | Q/L | L/L |
| Mutation combination 35 | D/S | L/GG | L/V | Q/I | H/L | L/I |
| Mutation combination 36 | D/T | V/N | L/I | N/I | N/L | L/V |
| Mutation combination 37 | E/S | I/N | I/L | Q/V | Q/I | I/L |
| Mutation combination 38 | E/T | L/N | I/V | N/V | H/I | I/I |
| Mutation combination 39 | N/S | V/GG | I/I | Q/L | N/I | I/L |
| Mutation combination 40 | N/T | I/GG | V/L | N/L | Q/V | V/L |
| Mutation combination 41 | D/S | L/GG | V/V | Q/I | H/V | V/I |
| Mutation combination 42 | D/T | V/N | V/I | N/I | N/V | V/V |
| Mutation combination 43 | E/S | I/N | L/L | Q/V | Q/L | L/L |
| Mutation combination 44 | E/T | L/N | L/V | N/V | H/L | L/I |
| Mutation combination 45 | N/S | V/GG | L/I | Q/L | N/L | L/V |
| Mutation combination 46 | N/T | I/GG | I/L | N/L | N/V | I/L |
| Mutation combination 47 | D/S | L/GG | I/V | Q/I | N/I | I/I |
| Mutation combination 48 | D/T | V/N | I/I | N/I | N/L | I/V |
| Mutation combination 49 | E/S | I/N | V/L | Q/V | Q/V | V/L |
| Mutation combination 50 | E/T | L/N | V/V | N/V | Q/I | V/I |
| Mutation combination 51 | N/S | V/GG | V/I | Q/L | Q/L | V/V |
| Mutation combination 52 | N/T | I/GG | L/L | N/L | H/V | L/L |
| Mutation combination 53 | E/T | L/GG | L/V | Q/I | H/I | L/I |
| Mutation combination 54 | N/S | I/N | L/I | N/I | H/L | L/V |
| Mutation combination 55 | N/T | L/N | I/L | Q/V | N/V | I/V |
| Mutation combination 56 | D/S | V/GG | I/V | N/V | N/I | I/I |
| Mutation combination 57 | D/T | I/GG | I/I | Q/L | N/L | I/L |
| Mutation combination 58 | E/S | V/N | V/L | N/L | Q/V | V/V |
| Mutation combination 59 | E/T | I/N | V/V | Q/I | Q/I | V/I |
| Mutation combination 60 | N/S | L/N | V/I | N/I | Q/L | V/L |
| Mutation combination 61 | N/T | V/GG | L/L | Q/I | H/V | L/V |

In one or more embodiments, X1s present in the six CDRs of the binding protein described in the present disclosure each independently represent the amino acid defined in the present disclosure; X2s present in the six CDRs of the binding protein described in the present disclosure each independently represent the amino acid defined in the present disclosure; X3s present in the six CDRs of the binding protein described in the present disclosure each independently represent the amino acid defined in the present disclosure.

In one or more embodiments, the binding protein includes at least 3 CDRs; alternatively, the binding protein includes at least 6 CDRs.

In one or more embodiments, the binding protein is an intact antibody including a variable region and a constant region. In one or more embodiments, the binding protein is one of a nanobody, F(ab')2, Fab', Fab, Fv, scFv, a bispecific antibody and a minimal recognition unit of antibody.

In one or more embodiments, the binding protein include light chain framework regions FR-L1, FR-L2, FR -L3 and FR-L4 with sequences correspondingly shown in SEQ ID NO: 1-4, and/or, heavy chain framework regions FR-H1, FR-H2, FR-H3 and FR-H4 with sequences correspondingly shown in SEQ ID NO: 5-8.

In one or more embodiments, the binding protein further includes an antibody constant region sequence.

In one or more embodiments, the constant region sequence is a sequence of any one constant region selected from IgG1, IgG2, IgG3, IgG4, IgA, IgM, IgE, and IgD.

In one or more embodiments, the constant region is derived from species consisted of cattle, horse, dairy cow, pig, sheep, goat, rat, mouse, dog, cat, rabbit, camel, donkey, deer, mink, chicken, duck, goose, turkey, gamecock or human. In one or more embodiments, the constant region is derived from the mouse;
a light chain constant region sequence is shown in SEQ ID NO: 9;
a heavy chain constant region sequence is shown in SEQ ID NO: 10.

The present disclosure also provides an isolated nucleic acid, encoding the above binding protein.

A nucleic acid herein includes a conservatively substituted variant thereof (for example, substitution of degenerate codons) and a complementary sequence. The terms "nucleic acid" and "polynucleotide" are synonymous and include a gene, cDNA molecule, mRNA molecule and their fragment such as oligonucleotide.

The present disclosure also provides a vector, including the above nucleic acid.

The nucleic acid sequence therein is operably linked to at least one regulatory sequence. "Operably linked" means that a coding sequence is linked to a regulatory sequence in a manner that allows the expression of the coding sequence. Regulatory sequence selection is used to direct the expression of a target protein in a suitable host cell, including a promoter, enhancer and other expression control elements.

A vector herein may refer to a molecule or agent that contains the nucleic acid of the present disclosure or a fragment thereof, can carry genetic information and can deliver genetic information into a cell. Typical vector includes a plasmid, virus, bacteriophage, cosmid, and mini-chromosome. The vector can be a cloning vector (that is, a vector used to transfer genetic information into a cell that can be propagated and selected by presence or absence of the genetic information) or an expression vector (that is, a vector containing necessary genetic elements to allow the genetic information of the vector to be expressed in a cell). Therefore, the cloning vector may include a selection marker and an origin of replication that matches the cell type specified by the cloning vector, and the expression vector may include regulatory elements necessary for affecting expression in the specified target cell.

The nucleic acid of the present disclosure or a fragment thereof can be inserted into a suitable vector to form a cloning vector or an expression vector carrying the nucleic acid fragment of the present disclosure. This new vector is also as part of this disclosure. The vector may include a plasmid, phage, cosmid, mini-chromosome or virus, as well as naked DNA that is only transiently expressed in specific cells. The cloning vector and expression vector of the present disclosure can replicate spontaneously, and therefore can provide a high copy number for the purpose of high-level expression or high-level replication for subsequent cloning. The expression vector may include a promoter for driving the expression of the nucleic acid fragment of the present disclosure, optionally a nucleic acid sequence encoding a signal peptide that allows the peptide expression product to be secreted or integrated into the membrane, the nucleic acid fragment of the present disclosure, and optionally a nucleic acid sequence encoding a terminator. When the expression vector is operated in the production strain or cell strain, the vector may be integrated into the genome of the host cell when it is introduced into the host cell, or it may not be integrated into the genome of the host cell. A vector usually carries a replication site and marker sequence that can provide phenotypic selection in a transformed cell.

The expression vector of the present disclosure is used to transform a host cell. Such transformed cell is also as part of the present disclosure, and may be a cultured cell or cell strain used to propagate the nucleic acid fragment and vector of the present disclosure, or to recombinantly prepare the polypeptide of the present disclosure. The transformed cell of the present disclosure includes microorganisms such as bacteria (such as *Escherichia coli, Bacillus,* etc.). The host cell also includes cells from multicellular organisms such as fungi, insect cells, plant cells or mammalian cells, preferably cells from mammals, such as CHO cells. The transformed cell is capable of replicating the nucleic acid fragment of the present disclosure. When the peptide combination of the present disclosure is recombinantly prepared, the expression product can be exported to the culture medium or carried on the surface of the transformed cell.

The present disclosure also provides a method for producing the above binding protein, including the steps of:
culturing the above host cell in a culture medium, recovering a produced binding protein from the culture medium or from the cultured host cell.

The method can be, for example, transfecting a host cell with a nucleic acid vector encoding at least a part of the binding protein, and culturing the host cell under suitable conditions to express the binding protein. The host cell can also be transfected with one or more expression vectors, which alone or in combination can contain DNA encoding at least a part of the binding protein. The binding protein can be separated from the culture medium or cell lysates using conventional techniques, including ammonium sulfate precipitation, chromatography (such as ion exchange, gel filtration, affinity chromatography, etc.) and/or electrophoresis, for purifying proteins and peptides.

The construction of a suitable vector containing the coding and regulatory sequences of interest can be carried out using standard ligation and restriction techniques well known in the art. The isolated plasmids, DNA sequences or synthetic oligonucleotides are cut, tailed and religated as required. Any method may be used to introduce the mutation into the coding sequence to produce the variant of the present disclosure, and such mutation may include deletion, insertion or substitution and the like.

The present disclosure also provides an antibody that can react with the epitope of Taq DNA polymerase, including monoclonal and polyclonal antibody. The antibody may contain an intact binding protein, or a fragment or derivative thereof. Preferred antibody contains all or part of the binding protein.

The present disclosure also provides a use of the above binding protein in PCR.

The present disclosure also provides a kit, including one or more of the above binding proteins, the above isolated nucleic acid, or the above vector.

The present disclosure also provides a composition, including the binding protein of the present disclosure and a Taq DNA polymerase.

In one or more embodiments, the composition further includs 4 deoxynucleoside triphosphates.

In one or more embodiments, the composition further includes a primer and/or a probe.

In one or more embodiments, the composition further includes MgCl₂.

In one or more embodiments, the composition further includes a nucleic acid as a template.

The present disclosure also provides a method for amplifying nucleic acid, including carrying out hot start PCR by using the binding protein of the present disclosure or the composition of the present disclosure.

In one or more embodiments, the hot start PCR is selected from a group consisted of multiplex PCR, real-time PCR, and real-time quantitative PCR. As used herein, the term "hot start PCR refers to PCR that makes Taq DNA polymerase work only when the sample temperature exceeds a certain temperature, thereby improving the specificity of the reaction and avoiding non-specific amplification of nucleic acids.

The present disclosure also provides a method for detecting Taq DNA polymerase in a test sample, incluing the steps of:
a) contacting the Taq DNA polymerase in the test sample with the binding protein of the present disclosure to form an immune complex under conditions sufficient for an antibody/antigen binding reaction to occur; and
b) detecting the presence of the immune complex, which indicates the presence of the Taq DNA polymerase in the test sample.

In one or more embodiments, the immune complex further includes a second antibody that binds to the binding protein;

In one or more embodiments, in step a), the immune complex further includes a second antibody that binds to the Taq DNA polymerase.

The present disclosure also provides use of the binding protein described herein in amplifying a nucleic acid.

Some examples are provided below to illustrate the present disclosure, but not to limit the scope of the present disclosure.

### Example 1

In this example, the restriction endonuclease and Prime Star DNA polymerase were purchased from Takara Company. The MagExtractor-RNA extraction kit was purchased from TOYOBO Company. SMARTERTM RACE cDNA Amplification Kit was purchased from Takara Company. The pMD-18T vector was purchased from Takara Company. The plasmid extraction kit was purchased from Tiangen Company. The primer synthesis and gene sequencing were completed by Invitrogen Company. The hybridoma cell strain that secreted the Anti-TAQ 2C7 monoclonal antibody was an existing hybridoma cell strain and was resuscitated for use.

### 1. Primer

Amplification of 5'RACE primers for heavy chains and light chains:
SMARTER IIA oligonucleotide:
5'-AAGCAGTGGTATCAACGCAGAGTACXXXXX-3';
5'-RACE CDS Primer (5'-CDS): 5'-(T)₂₅VN-3'(N=A,C,G or T; V=A,G, or C);
Universal primer A mixture (UPM): 5'-CTAATACGACTCACTATAGGGCAAGCAGTGGTATCAACGCAGA GT-3';
Nested universal primer A (NUP): 5'-AAGCAGTGGTATCAACGCAGAGT-3';
mlg-KR: 5 '-CTAACACTCATTCCTGTTGAAGCTCTTGACAAT-3';
mlg-HR: 5 '-TCATTTACCAGGAGAGTGGGAGAGGC-3'.

### 2. Gene cloning and sequencing for antibody variable region

The RNA was extracted from the hybridoma cell strain secreting the Anti-Taq 2C7 clone antibody, and the first chain cDNA was synthesized using the SMARTER™ RACE cDNA Amplification Kit and the SMARTER IIA oligonucleotide and 5'-CDS primers in the kit. The obtained first chain cDNA product was used as a template for PCR amplification. Light chain genes were amplified with universal primer A mix (UPM), nested universal primer A (NUP) and mKR primer, and heavy chain genes were amplified with universal primer A mix (UPM), nested universal primer A (NUP) and mHR primer. Wherein, the primer pair for the light chain amplified the target band of about 0.7 KB, and the primer pair for the heavy chain amplified the target band of about 1.4 KB. The product purified and recovered by agarose gel electrophoresis was subjected to A addition reaction with rTaq DNA polymerase, followed by inserting into pMD-18T vector and transforming into DH5 α competent cells. After the growth of the colony, 4 clones of the heavy chain and light chain gene clones were picked and sent to Invitrogen Company for sequencing.

### 3. Sequence analysis of variable region genes of Anti-Taq 2C7 antibody

The gene sequence obtained by the above sequencing was analyzed in the IMGT antibody database, and the amplified genes were analyzed to confirm that the heavy chain and light chain primers were correct, and the light chain amplified genes by using VNTI11.5 software. Among the gene fragments amplified by the light chain, the VL gene sequence was 378 bp, belonging to the Vkll gene family, with a 57bp leader peptide sequence in front of it; among the gene fragments amplified by the heavy chain primer pair, the VH gene sequence was 417bp, belonging to the VH1 gene family, with a 57 bp leader peptide sequence in front of it.

### 4. Construction of recombinant antibody expression plasmid

pcDNA™ 3.4 TOPO® vector was a constructed recombinant antibody eukaryotic expression vector. The expression vector had been introduced with HindIII, BamHI, EcoRI and other polyclonal restriction sites, named as pcDNA3.4A expression vector, and then referred to as 3.4A expression vector; according to the above sequencing results of antibody variable region genes in pMD-18T, the VL and VH gene-specific primers of Anti-TAQ 2C7 antibody were designed, with HindIII and EcoRI restriction sites and protective bases at both terminals, with the primers as follows:
Anti-Taq 2C7-HF: 5'-CCCAAGCTTGCCACCATGGGATGGAGCTATATCATCCTC-3';
Anti-Taq 2C7-HR:
   5'-CCCGAATTCTCATTATTTACCAGGAGAGTGGGAGAGGCTCTTCTC-3';
Anti- Taq 2C7-LF: 5'-CCCAAGCTTGCCACCATGTCCTCTGCTCAGTTCCTTGGTCTC-3';
Anti- Taq 2C7-LR:
   5'-CCCGAATTCTCATTAACACTCATTCCTGTTGAAGCTCTTGACAA-3'.

The 0.75KB light chain gene fragment and 1.42KB heavy chain gene fragment were amplified by PCR amplification method. The heavy chain and light chain gene fragments were digested with HindIII/EcoRI, respectively, and the 3.4A vector was digested with HindIII/EcoRI. After the fragment and vector being purified and recovered, the heavy chain gene and light chain gene were linked to the 3.4A expression vector, respectively, to obtain recombinant expression plasmids of heavy chain and light chain.

### 5. Screening of stable cell strains

### 5.1 Recombinant antibody expression plasmid was transiently transfected into CHO cells to confirm the activity

Plasmids were diluted with ultrapure water to 400 ng/ml, and the CHO cells were adjusted to 1.43×10⁷cells/ml in a centrifuge tube. 100µl of plasmids were mixed with 700µl of cells, which were transferred to the electrotransfection cuvette for electrotransfection. The samples were taken and counted on Day 3, 5 and 7, and were collected on Day 7 for testing.

Taq enzyme was diluted with coating solution to the specified concentration at 100 µL per well overnight at 4°C; which was washed twice with washing solution and patted dry on the next day; with addition of blocking solution (20%BSA+80%PBS) at 120 µL per well at 37°C for 1 h and patted dry; added the diluted cell supernatant, 100 µL/well at 37°C for 30 min (partial supernatant for 1 h); washed 5 times with washing solution and patted dry; added goat anti-mouse IgG-HRP 100 µL per well at 37°C for 30 min; washed 5 times with washing solution and patted dry; added color developing solution A (50 µL/well), added color developing solution B (50 µL/well) for 10 min; added stop solution, 50µL/well; followed by reading the OD value at 450nm (reference 630 nm) on the microplate reader. The results showed that the OD of the reaction was still greater than 1.0 after the cell supernatant being diluted 1000 times, and the reaction OD of the wells without the cell supernatant was less than 0.1, indicating that the antibody produced after transient transfection with the plasmid was active against Taq enzyme.

### 5.2 Linearization of recombinant antibody expression plasmid

Preparation of the following reagents: Buffer 50 µl, DNA 100 µg/tube, Puv I enzyme 10 µl, supplemented with sterile water to 500 µl, for digestion overnight at 37°C in the water bath; which was extracted with an equal volume of phenol/chloroform/isoamyl alcohol (lower layer) 25:24:1 and then chloroform (aqueous phase) in sequence; precipitated on ice with 0.1 times volume (aqueous phase) 3M sodium acetate and 2 times volume of ethanol, and the obtained precipitate was rinsed with 70% ethanol to remove the organic solvent, followed by thawing with an appropriate amount of sterilized water until the ethanol evaporated completely to be determined the concentration finally.

### 5.3 Stable transfection of recombinant antibody expression plasmid and selection of stable cell strains under pressure

Plasmids were diluted with ultrapure water to 400 ng/ml, and the CHO cells were adjusted to 1.43×10⁷cells/ml in a centrifuge tube. 100µl of plasmids were mixed with 700µl of cells, which were transferred to the electrotransfection cuvette for electrotransfection and counted on the next day, followed by culturing at 25 µmol/L MSX 96 wells under pressure for about 25 days.

The clonal wells with labeled cells were observed under a microscope, and recorded the confluence; from which the culture supernatant were taken and sent for testing; and cell strains with high antibody concentration and relative concentration were transferred to 24 wells, and then transfered to 6 wells in about 3 days; followed by preservation and batch culture with adjustment the cell density to 0.5×10⁶cells/ml, from which 2.2 ml for batch culture at the cell density of 0.3×10⁶cells/ml, and 2 ml for preservation; and the supernatant from 6 wells batch culture for 7 days was sent for testing, from which the cell strain with smaller antibody concentration and cell diameter was selected for TPP preservation and passage.

### 6. Production of recombinant antibodies

### 6.1 Cell expansion culture

After resuscitation, the cells were cultured in a 125 ml shake flask with a 30 ml inoculation volume and 100% Dynamis medium, placed in a shaker with a rotation speed of 120 r/min at 37°C, and a carbon dioxide of 8%. Cells were cultured for 72 h and inoculated at an inoculation density of 500,000 cells/ml for expansion culture, in which the expansion volume was calculated according to production requirements, using 100% Dynamis medium. Then the culture was expanded every 72 h. When the cell mass met the production requirements, the inoculation density was strictly controlled to about 500,000 cells/ml for production.

### 6.2 Production in the shake flask and purification

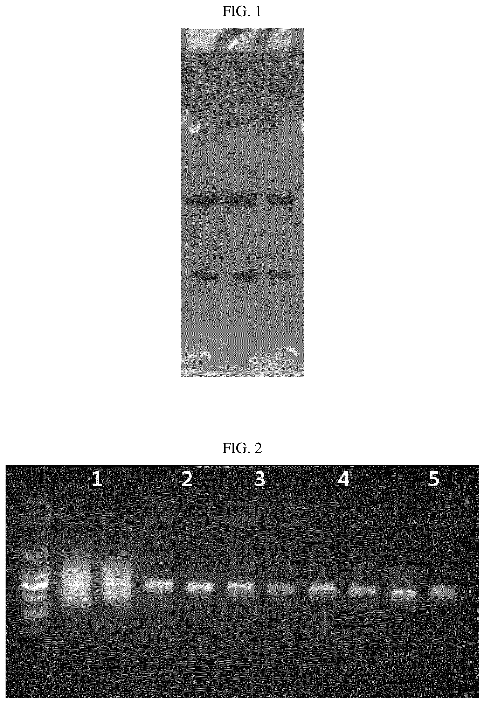

Shake flask parameters: rotation at a speed of 120 r/min, at a temperature of 37°C, and with carbon dioxide of 8%. Feeding: feeding every day was started at after it is cultured for 72 h in the shake flask. HyCloneTM Cell BoostTM Feed 7a was fed 3% of the initial culture volume every day, and Feed 7b with fed every thousandth of the initial culture volume, until to Day 12 (feeding on Day 12). Glucose was supplemented at 3g/L on Day 6. Samples were collected on Day 13. Then the affinity purification was carried out by a proteinA affinity chromatography column. 4 µg of purified antibodies was taken for reducibility SDS-PAGE, and 4 µg of foreign control antibodies was used as a control. The electrophoretogram was shown in FIG. 1. Two bands were shown after reducibility SDS-PAGE, in which one Mr was 50 KD (heavy chain), having the sequence shown in SEQ ID NO: 11; and the other Mr was 28 KD (light chain), having the sequence shown in SEQ ID NO: 12.

### Example 2

The antibody of sample 1 obtained in Example 1 (having the heavy chain and light chain shown in SEQ ID NO: 11 and 12) had the ability to bind to Taq DNA polymerase, but the affinity and antibody activity were not ideal, therefore, the applicant mutated the light chain CDR and heavy chain CDR of the antibody.

After analysis, in the complementarity determining region (WT) of the heavy chain:
CDR-VH1 was S-V-D (X1)-T-F-S (X2)-T-Y-Y-L (X3)-Y;
CDR-VH2 was G-V (X1)-N-P-T-S-N (X2)-P-V-F-D (X3)-E-K;
CDR-VH3 was T-R-S-I (X1)-L (X2)-R-R-G-Y-Y-P (X3)-D-Y;

In the complementarity determining region of the light chain:
CDR-VL1 was R-G (X1)-S-Q-D-I-Q (X2)-N-Y-V (X3)-N;
CDR-VL2 was I-Y-F (X1)-T-S-R-L-Q (X2)-S-G-I (X3)-P;
CDR-VL3 was Q-D-D-T-I (X1)-P-V (X2)-T-W (X3)-G;
wherein, X1, X2, and X3 were all mutation sites.

**Table 1 Mutation sites related to antibody activity**

| Site | CDR-VH1 X3 | CDR-VH2 X3 | CDR-VH3 X3 | CDR-VL1 X1 | CDR-VL2 X1 | CDR-VL3 X3 |
|---|---|---|---|---|---|---|
| WT | L | D | P | G | F | W |
| Mutation 1 | I | N | F | A | Y | F |
| Mutation 2 | I | N | P | G | F | F |
| Mutation 3 | A | D | Q | G | Y | Y |
| Mutation 4 | W | K | E | L | S | E |
| Mutation 5 | S | Y | K | I | P | R |

The antibody activty was detected after mutation. Taq DNA enzyme was diluted with coating solution to the specified concentration, 100 µL per well overnight at 4°C; which was washed twice with washing solution and patted dry on the next day; with addition of blocking solution (20%BSA+80%PBS), 120 µL per well at 37°C for 1 h and patted dry; added the diluted Taq monoclonal antibodies, 100 µL/well at 37°C for 30 min (partial supernatant for 1 h); washed 5 times with washing solution and patted dry; added goat anti-mouse IgG-HRP at 100 µL per well at 37°C for 30 min; washed 5 times with washing solution and patted dry; added color developing solution A (50 µL/well), added color developing solution B (50 µL/well) for 10 min; added stop solution, 50µL/well; followed by reading the OD value at 450nm (reference 630 nm) on the microplate reader.

Some of the results were as follows:

**Table 2 Antibody activity analysis data**

| Concentration (ng/ml) | WT | Mutation 1 | Mutation 2 | Mutation 3 | Mutation 4 | Mutation 5 |
|---|---|---|---|---|---|---|
| 37.04 | 2.298 | 2.382 | 2.054 | 1.784 | 1.378 | 1.405 |
| 12.35 | 2.101 | 2.139 | 1.841 | 0.874 | 0.679 | 0.701 |
| 4.12 | 1.578 | 1.697 | 1.005 | 0.450 | 0.354 | 0.362 |
| 1.37 | 0.798 | 0.887 | 0.514 | 0.067 | 0.057 | 0.054 |
| 0.46 | 0.399 | 0.442 | 0.059 | - | - | - |
| 0 | 0.035 | 0.047 | 0.050 | - | - | - |

| | | | | | | |
|---|---|---|---|---|---|---|
| "-" means no activity. | | | | | | |

### Affinity analysis

Using AMC sensor, the above antibody was diluted to 10 µg/ml with PBST, and Taq DNA polymerase was diluted with PBST at: 1000 nmol/ml, 500 nmol/ml, 250 nmol/ml, 125 nmol/ml, 62.5 nmol/ml, 31.3 nmol/ml, 15.6 nmol/ml, and 0 nmol/ml.

Running process: equilibration in buffer 1 (PBST) for 60 s, antibody immobilization in antibody solution for 300 s, incubation in buffer 2 (PBST) for 180 s, binding in the antigen solution for 420 s, dissociation in buffer 2 for 1200 s, regeneration the sensor with 10 mM pH 1.69 GLY solution and buffer 3 and data output. KD represented the equilibrium unaffinity constant or affinity; Kon represented the rate of binding; Kdis represents the rate of dissociation.

**Table 3 Affinity analysis data**

| Different mutations | KD(M) | Kon (1/Ms) | Kdis (1/S) |
|---|---|---|---|
| WT | 2.457E-09 | 5.904E+04 | 1.451E-04 |
| Mutation 1 | 3.839E-10 | 9.832E+04 | 3.775E-05 |
| Mutation 2 | 9.900E-10 | 5.924E+04 | 5.865E-05 |
| Mutation 3 | 2.066E-08 | 6.550E+04 | 1.353E-03 |
| Mutation 4 | | | |
| Mutation 5 | | | |

| | | | |
|---|---|---|---|
| "-" means not detected. | | | |

From Table 2 and Table 3, mutation 1 was used as the framework sequence to screen for mutation sites with better potency due to having best activity and affinity (ensure that the antibody activity obtained by screening was similar to that of mutation 1, having the antibody activity of ±10 %), some of the results were as follows.

**Table 4 Mutation sites related to antibody affinity**

| Site | CDR-VH1 X1/X2 | CDR-VH2 X1/X2 | CDR-VH3 X1/X2 | CDR-VL1 X2/X3 | CDR-VL2 X2/X3 | CDR-VL3 X1/X2 |
|---|---|---|---|---|---|---|
| Mutation 1 | D/S | V/N | I/L | Q/V | Q/I | I/V |
| Mutation 1-1 | D/T | I/N | I/V | N/V | Q/V | I/I |
| Mutation 1-2 | E/S | L/N | I/I | Q/L | Q/L | I/L |
| Mutation 1-3 | E/T | V/GG | V/L | N/L | H/I | V/V |
| Mutation 1-4 | N/S | I/GG | V/V | Q/I | H/V | V/I |
| Mutation 1-5 | N/T | L/GG | V/I | N/I | H/L | V/L |
| Mutation 1-6 | D/S | V/N | L/L | Q/V | N/I | L/V |
| Mutation 1-7 | D/T | I/N | L/V | N/V | N/V | L/I |
| Mutation 1-8 | E/S | L/N | L/I | Q/L | N/L | L/L |
| Mutation 1-9 | E/T | V/GG | I/L | N/L | Q/I | I/V |
| Mutation 1-10 | N/S | I/GG | I/V | Q/I | Q/V | I/I |
| Mutation 1-11 | N/T | L/GG | I/I | N/I | Q/L | I/L |
| Mutation 1-12 | D/S | V/N | V/L | Q/V | H/I | V/V |
| Mutation 1-13 | D/T | I/N | V/V | N/V | H/V | V/I |
| Mutation 1-14 | E/S | L/N | V/I | Q/L | H/L | V/L |
| Mutation 1-15 | E/T | V/GG | L/L | N/L | N/I | L/V |
| Mutation 1-16 | N/S | I/GG | L/V | Q/I | N/V | L/I |
| Mutation 1-17 | N/T | L/GG | L/I | N/I | N/L | L/L |
| Mutation 1-18 | D/T | V/N | I/L | Q/V | Q/I | I/V |
| Mutation 1-19 | E/S | I/N | I/V | N/V | H/I | I/I |
| Mutation 1-20 | E/T | L/N | I/I | Q/L | N/I | I/L |
| Mutation 1-21 | N/S | V/GG | V/L | N/L | Q/V | V/V |
| Mutation 1-22 | N/T | I/GG | V/V | Q/I | H/V | V/I |
| Mutation 1-23 | D/S | L/GG | V/I | N/I | N/V | V/L |
| Mutation 1-24 | E/S | V/GG | L/L | Q/V | Q/L | L/V |
| Mutation 1-25 | E/T | I/GG | L/V | N/V | H/L | L/I |
| Mutation 1-26 | N/S | L/GG | L/I | Q/L | N/L | L/L |
| Mutation 1-27 | N/T | V/N | I/L | N/L | Q/I | I/V |
| Mutation 148 | D/S | I/N | I/V | Q/I | H/I | I/I |
| Mutation 1-29 | E/T | L/N | I/I | N/I | N/I | I/V |
| Mutation 1-30 | N/S | I/GG | V/L | Q/V | Q/V | VL |
| Mutation 1-31 | N/T | L/GG | V/V | N/V | H/V | V/I |
| Mutation 1-32 | D/S | V/GG | V/I | Q/L | N/V | V/V |
| Mutation 1-33 | E/S | I/GG | L/L | N/L | Q/L | L/L |
| Mutation 1-34 | D/S | L/GG | L/V | Q/I | H/L | L/I |
| Mutation 1-35 | D/T | V/N | L/I | N/I | N/L | L/V |
| Mutation 1-36 | E/S | I/N | I/L | Q/V | Q/I | I/L |
| Mutation 1-37 | E/T | L/N | I/V | N/V | H/I | I/I |
| Mutation 1-38 | N/S | V/GG | I/I | Q/L | N/I | I/L |
| Mutation 1-39 | N/T | I/GG | V/L | N/L | Q/V | V/L |
| Mutation 1-40 | D/S | L/GG | V/V | Q/I | H/V | V/I |
| Mutation 1-41 | D/T | V/N | V/I | N/I | N/V | V/V |
| Mutation 1-42 | E/S | I/N | L/L | Q/V | Q/L | L/L |
| Mutation 1-43 | E/T | L/N | L/V | N/V | H/L | L/I |
| Mutation 1-44 | N/S | V/GG | L/I | Q/L | N/L | L/V |
| Mutation 1-45 | N/T | I/GG | I/L | N/L | N/V | I/L |
| Mutation 1-46 | D/S | L/GG | I/V | Q/I | N/I | I/I |
| Mutation 1-47 | D/T | V/N | I/I | N/I | N/L | I/V |
| Mutation 1-48 | E/S | I/N | V/L | Q/V | Q/V | V/L |
| Mutation 1-49 | E/T | L/N | V/V | N/V | Q/I | V/I |
| Mutation 1-50 | N/S | V/GG | V/I | Q/L | Q/L | V/V |
| Mutation 1-51 | N/T | I/GG | L/L | N/L | H/V | L/L |
| Mutation 1-52 | E/T | L/GG | L/V | Q/I | H/I | L/I |
| Mutation 1-53 | N/S | I/N | L/I | N/I | H/L | L/V |
| Mutation 1-54 | N/T | L/N | I/L | Q/V | N/V | I/V |
| Mutation 1-55 | D/S | V/GG | I/V | N/V | N/I | I/I |
| Mutation 1-56 | D/T | I/GG | I/I | Q/L | N/L | I/L |
| Mutation 1-57 | E/S | V/N | V/L | N/L | Q/V | V/V |
| Mutation 1-58 | E/T | I/N | V/V | Q/I | Q/I | V/I |
| Mutation 1-59 | N/S | L/N | V/I | N/I | Q/L | V/L |
| Mutation 1-60 | N/T | V/GG | L/L | Q/I | H/V | L/V |

The method of affinity analysis was as above, and the results were shown in Table 5.

**Table 5 Affinity analysis data**

| Different mutations | KD (M) | Kon (1/Ms) | Kdis (1/S) |
|---|---|---|---|
| Mutation 1 | 3.839E-10 | 9.832E+04 | 3.775E-05 |
| Mutation 1-1 | 4.914E-10 | 3.217E+04 | 1.581E-05 |
| Mutation 1-2 | 2.850E-10 | 4.971E+04 | 1.417E-05 |
| Mutation 1-3 | 3.675E-10 | 4.196E+04 | 1.542E-05 |
| Mutation 1-4 | 7.192E-10 | 3.821E+04 | 2.748E-05 |
| Mutation 1-5 | 8.734E-10 | 8.910E+04 | 7.782E-05 |
| Mutation 1-6 | 8.085E-10 | 4.213E+04 | 3.406E-05 |
| Mutation 1-7 | 5.986E-10 | 4.100E+04 | 2.454E-05 |
| Mutation 1-8 | 3.435E-11 | 4.763E+04 | 1.636E-06 |
| Mutation 1-9 | 9.261E-10 | 7.865E+04 | 7.284E-05 |
| Mutation 1-10 | 3.839E-10 | 9.801E+04 | 3.764E-05 |
| Mutation 1-11 | 6.126E-10 | 4.999E+04 | 3.062E-05 |
| Mutation 1-12 | 2.196E-11 | 9.338E+04 | 2.051E-06 |
| Mutation 1-13 | 4.075E-10 | 6.174E+04 | 2.516E-05 |
| Mutation 1-14 | 2.286E-10 | 8.407E+04 | 1.922E-05 |
| Mutation 1-15 | 1.201E-10 | 4.760E+04 | 5.717E-06 |
| Mutation 1-16 | 2.227E-10 | 6.514E+04 | 1.451E-05 |
| Mutation 1-17 | 3.980E-10 | 3.405E+04 | 1.355E-05 |
| Mutation 1-18 | 6.772E-10 | 6.640E+04 | 4.497E-05 |
| Mutation 1-19 | 2.913E-10 | 6.333E+04 | 1.845E-05 |
| Mutation 1-20 | 7.281E-10 | 8.832E+04 | 6.430E-05 |
| Mutation 1-21 | 6.643E-11 | 8.978E+04 | 5.964E-06 |
| Mutation 1-22 | 3.839E-10 | 9.721E+04 | 3.698E-05 |
| Mutation 1-23 | 9.131E-10 | 7.022E+04 | 6.411E-05 |
| Mutation 1-24 | 1.989E-10 | 5.645E+04 | 1. 123E-05 |
| Mutation 1-25 | 6.784E-10 | 6.733E+04 | 4.568E-05 |
| Mutation 1-26 | 8.518E-10 | 3.397E+04 | 2.894E-05 |
| Mutation 1-27 | 2.805E-11 | 7.242E+04 | 2.031E-06 |
| Mutation 1-28 | 4.018E-10 | 6.498E+04 | 2.611E-05 |
| Mutation 1-29 | 3.145E-11 | 3.609E+04 | 1.135E-06 |
| Mutation 1-30 | 9.113E-10 | 4.927E+04 | 4.490E-05 |
| Mutation 1-31 | 8.499E-10 | 6.329E+04 | 5.379E-05 |
| Mutation 1-32 | 2.810E-10 | 3.132E+04 | 8.800E-06 |
| Mutation 1-33 | 9.259E-10 | 4.261E+04 | 3.945E-05 |
| Mutation 1-34 | 9.870E-10 | 6.255E+04 | 6.174E-05 |
| Mutation 1-35 | 7.055E-10 | 5.244E+04 | 3.700E-05 |
| Mutation 1-36 | 7.678E-10 | 5.018E+04 | 3.853E-05 |
| Mutation 1-37 | 3.820E-10 | 7.021E+04 | 2.682E-05 |
| Mutation 1-38 | 1.060E-10 | 3.136E+04 | 3.324E-06 |
| Mutation 1-39 | 4.574E-10 | 4.814E+04 | 2.202E-05 |
| Mutation 1-40 | 6.154E-10 | 7.645E+04 | 4.705E-05 |
| Mutation 1-41 | 2.968E-10 | 3.308E+04 | 9.819E-06 |
| Mutation 1-42 | 8.030E-10 | 4.673E+04 | 3.752E-05 |
| Mutation 1-43 | 9.713E-10 | 7.125E+04 | 6.920E-05 |
| Mutation 1-44 | 1.328E-11 | 3.690E+05 | 4.899E-06 |
| Mutation 1-45 | 5.824E-11 | 5.265E+04 | 3.066E-06 |
| Mutation 1-46 | 6.547E-10 | 4.377E+04 | 2.866E-05 |
| Mutation 1-47 | 7.394E-10 | 6.320E+04 | 4.673E-05 |
| Mutation 1-48 | 3.839E-10 | 9.792E+04 | 3.721E-05 |
| Mutation 1-49 | 1.983E-10 | 8.610E+04 | 1.708E-05 |
| Mutation 1-50 | 5.929E-10 | 5.085E+04 | 3.015E-05 |
| Mutation 1-51 | 3.836E-10 | 9.830E+04 | 3.771E-05 |
| Mutation 1-52 | 6.981E-10 | 3.398E+04 | 2.372E-05 |
| Mutation 1-53 | 3.839E-10 | 9.897E+04 | 3.801E-05 |
| Mutation 1-54 | 2.727E-10 | 3.723E+04 | 1.015E-05 |
| Mutation 1-55 | 1.734E-10 | 6.444E+04 | 1.117E-05 |
| Mutation 1-56 | 9.136E-10 | 4.148E+04 | 3.790E-05 |
| Mutation 1-57 | 3.616E-10 | 6.897E+04 | 2.494E-05 |
| Mutation 1-58 | 1.785E-10 | 7.498E+04 | 1.338E-05 |
| Mutation 1-59 | 5.067E-11 | 6.043E+04 | 3.062E-06 |
| Mutation 1-60 | 3.140E-11 | 8.461E+04 | 2.657E-06 |

From Table 5, the mutation sites listed in Table 4 had little effect on the affinity of the antibody.

In order to verify the above results, the above experiment was repeated with WT as the framework sequence to verify the affinity of the mutation site, with some of the results as follows.

**Table 6 Mutations with WT as the framework**

| Site | CDR-VH1 X1/X2 | CDR-VH2 X1/X2 | CDR-VH3 X1/X2 | CDR-VL1 X2/X3 | CDR-VL2 X2/X3 | CDR-VL3 X1/X2 |
|---|---|---|---|---|---|---|
| WT | D/S | V/N | I/L | Q/V | Q/I | I/V |
| WT 1-1 | D/T | I/N | I/V | N/V | Q/V | I/I |
| WT 1-2 | E/S | L/N | I/I | Q/L | Q/L | I/L |
| WT 1-3 | E/T | V/GG | V/L | N/L | H/I | V/V |
| WT 1-4 | N/S | I/GG | V/V | Q/I | H/V | V/I |
| WT1-5 | N/T | L/GG | V/I | N/I | H/L | V/L |
| WT 1-6 | D/S | V/N | L/L | Q/V | N/I | L/V |
| WT 1-7 | D/T | I/N | L/V | N/V | N/V | L/I |
| WT 1-8 | E/S | L/N | L/I | Q/L | N/L | L/L |
| WT 1-9 | E/T | V/GG | I/L | N/L | Q/I | I/V |
| WT 1-10 | N/S | I/GG | I/V | Q/I | Q/V | I/I |

**Table 7 Affinity analysis data**

| Different mutations | KD(M) | Kon (1/Ms) | Kdis (1/S) |
|---|---|---|---|
| WT | 2.457E-09 | 5.904E+04 | 1.451E-04 |
| WT 1-1 | 4.652E-09 | 5.879E+04 | 2.735E-04 |
| WT 1-2 | 3.414E-09 | 4.849E+04 | 1.655E-04 |
| WT 1-3 | 2.296E-09 | 4.802E+04 | 1.103E-04 |
| WT 1-4 | 4.100E-09 | 8.842E+04 | 3.625E-04 |
| WT 1-5 | 5.126E-09 | 6.125E+04 | 3.139E-04 |
| WT 1-6 | 6.652E-09 | 7.610E+04 | 5.062E-04 |
| WT 1-7 | 3.418E-09 | 8.668E+04 | 2.962E-04 |
| WT 1-8 | 3.018E-09 | 3.265E+04 | 9.855E-05 |
| WT 1-9 | 8.568E-09 | 5.054E+04 | 4.330E-04 |

From the analysis in Table 6 and 7, the mutation sites listed in Table 6 also had little effect on the affinity of the antibody.

The antibody of the present disclosure that was modified with Taq DNA polymerase, had an enzyme blocking activity below 70°C, could be dissociated above 70°C, and could be completely dissociated only 1-3 min at 95°C to release the active ingredient of the enzyme.

Under the equal conditions, the Taq DNA polymerase with and without antibody modification was detected specifically, with the results shown in the PCR electrophoretogram in FIG. 2, wherein 1 was Taq DNA polymerase (without antibody modification), 2 and 4 were Taq DNA polymerases modified by mutation 1 antibody, 3 and 5 were Taq DNA polymerases modified by WT antibody. The analysis of the results showed that the TAQ enzyme modified by the antibody had significantly improved amplification specificity. By analyzing the non-specific bands in each lane, it could find that the specificity of Taq DNA polymerase modified with mutation 1 antibody was slightly better than that of Taq DNA polymerase modified with WT antibody. The present disclosure also detected the binding stability, rapid activation, and pH compatibility of the antibody and Taq DNA polymerase on the mutation combinations in Table 4, all of which had excellent effects, indicating that the antibody which fought for Taq DNA polymerase provided by the present disclosure had a good application in molecular detection.

Finally, it should be noted that the above embodiments are only used to illustrate the technical solutions of the present disclosure, not to limit them; although the present disclosure has been described in detail with reference to the foregoing embodiments, those of ordinary skill in the art should understand that: the technical solutions recorded in the foregoing embodiments can still be modified, or some or all of the technical features can be equivalently replaced; and these modifications or replacements do not cause the essence of the corresponding technical solutions to deviate from the scope of the technical solutions of the embodiments in the present disclosure.

### Industrial Applicability

The binding protein provided in the present disclosure can specifically bind to Taq enzyme to form an abzyme complex, so it can effectively block the activity of Taq DNA polymerase at room temperature; while at high temperature, such complex will dissociate and release the active Taq DNA polymerase to perform PCR amplification reactions. This can effectively avoid the formation of primer dimers, reduce the amplification of non-specific products, and improve the long-term stability of Taq DNA polymerase. The binding protein of the present disclosure can be widely used in various hot start PCRs.

## Claims

1. An isolated binding protein comprising an antigen-binding domain, wherein the antigen-binding domain comprises at least one complementarity determining region selected from the following amino acid sequences, or has at least 80% sequence identity with the complementarity determining region of the following amino acid sequences and has an affinity of K_{D}≤8.568×10⁻⁹mol/L to a Taq DNA polymerase;
the complementarity determining region CDR-VH1 is S-V-X1-T-F-X2-T-Y-Y-X3-Y, wherein
X1 is D, E or N, X2 is S or T, and X3 is I or L;
the complementarity determining region CDR-VH2 is G-X1-N-P-T-S-X2-P-V-F-X3-E-K, wherein
X1 is I, V or L, X2 is N or GG, and X3 is D, E or N;
the complementarity determining region CDR-VH3 is T-R-S-X1-X2-R-R-G-Y-Y-X3-D-Y, wherein
X1 is I, V or L, X2 is I, V or L, and X3 is F or P;
the complementarity determining region CDR-VL1 is R-X1-S-Q-D-I-X2-N-Y-X3-N, wherein
X1 is A or G, X2 is N or Q, and X3 is I, V or L;
the complementarity determining region CDR-VL2 is I-Y-X1-T-S-R-L-X2-S-G-X3-P, wherein
X1 is Y or F, X2 is Q, H or N, and X3 is I, V or L;
the complementarity determining region CDR-VL3 is Q-D-D-T-X1-P-X2-T-X3-G, wherein
X1 is I, V or L, X2 is I, V or L, and X3 is W or F.

2. The binding protein according to claim 1, wherein
X3 is I in the complementarity determining region CDR-VH1;
X3 is N in the complementarity determining region CDR-VH2;
X3 is F in the complementarity determining region CDR-VH3;
X1 is A in the complementarity determining region CDR-VL1;
X1 is Y in the complementarity determining region CDR-VL2;
X3 is F in the complementarity determining region CDR-VL3;
further, X1 is D and X2 is S in the complementarity determining region CDR-VH1;
further, X1 is E and X2 is S in the complementarity determining region CDR-VH1;
further, X1 is N and X2 is S in the complementarity determining region CDR-VH1;
further, X1 is D and X2 is T in the complementarity determining region CDR-VH1;
further, X1 is E and X2 is T in the complementarity determining region CDR-VH1;
further, X1 is N and X2 is T in the complementarity determining region CDR-VH1;
further, X1 is I and X2 is N in the complementarity determining region CDR-VH2;
further, X1 is I and X2 is GG in the complementarity determining region CDR-VH2;
further, X1 is V and X2 is N in the complementarity determining region CDR-VH2;
further, X1 is V and X2 is GG in the complementarity determining region CDR-VH2;
further, X1 is L and X2 is N in the complementarity determining region CDR-VH2;
further, X1 is L and X2 is GG in the complementarity determining region CDR-VH2;
further, X1 is I and X2 is I in the complementarity determining region CDR-VH3;
further, X1 is I and X2 is V in the complementarity determining region CDR-VH3;
further, X1 is I and X2 is L in the complementarity determining region CDR-VH3;
further, X1 is V and X2 is I in the complementarity determining region CDR-VH3;
further, X1 is V and X2 is V in the complementarity determining region CDR-VH3;
further, X1 is V and X2 is L in the complementarity determining region CDR-VH3;
further, X1 is L and X2 is I in the complementarity determining region CDR-VH3;
further, X1 is L and X2 is V in the complementarity determining region CDR-VH3;
further, X1 is L and X2 is L in the complementarity determining region CDR-VH3;
further, X2 is N and X3 is I in the complementarity determining region CDR-VL1;
further, X2 is N and X3 is V in the complementarity determining region CDR-VL1;
further, X2 is N and X3 is L in the complementarity determining region CDR-VL1;
further, X2 is Q and X3 is I in the complementarity determining region CDR-VL1;
further, X2 is Q, and X3 is V in the complementarity determining region CDR-VL1;
further, X2 is Q and X3 is L in the complementarity determining region CDR-VL1;
further, X2 is Q and X3 is I in the complementarity determining region CDR-VL2;
further, X2 is Q and X3 is V in the complementarity determining region CDR-VL2;
further, X2 is Q and X3 is L in the complementarity determining region CDR-VL2;
further, X2 is H and X3 is I in the complementarity determining region CDR-VL2;
further, X2 is H and X3 is V in the complementarity determining region CDR-VL2;
further, X2 is H and X3 is L in the complementarity determining region CDR-VL2;
further, X2 is N and X3 is I in the complementarity determining region CDR-VL2;
further, X2 is N and X3 is V in the complementarity determining region CDR-VL2;
further, X2 is N and X3 is L in the complementarity determining region CDR-VL2;
further, X1 is I and X2 is I in the complementarity determining region CDR-VL3;
further, X1 is I and X2 is V in the complementarity determining region CDR-VL3;
further, X1 is I and X2 is L in the complementarity determining region CDR-VL3;
further, X1 is V and X2 is I in the complementarity determining region CDR-VL3;
further, X1 is V and X2 is V in the complementarity determining region CDR-VL3;
further, X1 is V and X2 is L in the complementarity determining region CDR-VL3;
further, X1 is L and X2 is I in the complementarity determining region CDR-VL3;
further, X1 is L and X2 is V in the complementarity determining region CDR-VL3;
further, X1 is L and X2 is L in the complementarity determining region CDR-VL3;
preferably, a mutation site of each complementarity determining region is selected from any one of the following mutation combinations:
| Site | CDR-VH1 X1/X2 | CDR-VH2 X1/X2 | CDR-VH3 X1/X2 | CDR-VL 1 X2/X3 | CDR-VL2 X2/X3 | CDR-VL3 X1/X2 |
|---|---|---|---|---|---|---|
| Mutation combination 1 | D/S | V/N | I/L | Q/V | Q/I | I/V |
| Mutation combination 2 | D/T | I/N | I/V | N/V | Q/V | I/I |
| Mutation combination 3 | E/S | L/N | I/I | Q/L | Q/L | I/L |
| Mutation combination 4 | E/T | V/GG | V/L | N/L | H/I | V/V |
| Mutation combination 5 | N/S | I/GG | V/V | Q/I | H/V | V/I |
| Mutation combination 6 | N/T | L/GG | V/I | N/I | H/L | V/L |
| Mutation combination 7 | D/S | V/N | L/L | Q/V | N/I | L/V |
| Mutation combination 8 | D/T | I/N | L/V | N/V | N/V | L/I |
| Mutation combination 9 | E/S | L/N | L/I | Q/L | N/L | L/L |
| Mutation combination 10 | E/T | V/GG | I/L | N/L | Q/I | I/V |
| Mutation combination 11 | N/S | I/GG | I/V | Q/I | Q/V | I/I |
| Mutation combination 12 | N/T | L/GG | I/I | N/I | Q/L | I/L |
| Mutation combination 13 | D/S | V/N | V/L | Q/V | H/I | V/V |
| Mutation combination 14 | D/T | I/N | V/V | N/V | H/V | V/I |
| Mutation combination 15 | E/S | L/N | V/I | Q/L | H/L | V/L |
| Mutation combination 16 | E/T | V/GG | L/L | N/L | N/I | L/V |
| Mutation combination 17 | N/S | I/GG | L/V | Q/I | N/V | L/I |
| Mutation combination 18 | N/T | L/GG | L/I | N/I | N/L | L/L |
| Mutation combination 19 | D/T | V/N | I/L | Q/V | Q/I | I/V |
| Mutation combination 20 | E/S | I/N | I/V | N/V | H/I | I/I |
| Mutation combination 21 | E/T | L/N | I/I | Q/L | N/I | I/L |
| Mutation combination 22 | N/S | V/GG | V/L | N/L | Q/V | V/V |
| Mutation combination 23 | N/T | I/GG | V/V | Q/I | H/V | V/I |
| Mutation combination 24 | D/S | L/GG | V/I | N/I | N/V | V/L |
| Mutation combination 25 | E/S | V/GG | L/L | Q/V | Q/L | L/V |
| Mutation combination 26 | E/T | I/GG | L/V | N/V | H/L | L/I |
| Mutation combination 27 | N/S | L/GG | L/I | Q/L | N/L | L/L |
| Mutation combination 28 | N/T | V/N | I/L | N/L | Q/I | I/V |
| Mutation combination 29 | D/S | I/N | I/V | Q/I | H/I | I/I |
| Mutation combination 30 | E/T | L/N | I/I | N/I | N/I | I/V |
| Mutation combination 31 | N/S | I/GG | V/L | Q/V | Q/V | VL |
| Mutation combination 32 | N/T | L/GG | V/V | N/V | H/V | V/I |
| Mutation combination 33 | D/S | V/GG | V/I | Q/L | N/V | V/V |
| Mutation combination 34 | E/S | I/GG | L/L | N/L | Q/L | L/L |
| Mutation combination 35 | D/S | L/GG | L/V | Q/I | H/L | L/I |
| Mutation combination 36 | D/T | V/N | L/I | N/I | N/L | L/V |
| Mutation combination 37 | E/S | I/N | I/L | Q/V | Q/I | I/L |
| Mutation combination 38 | E/T | L/N | I/V | N/V | H/I | I/I |
| Mutation combination 39 | N/S | V/GG | I/I | Q/L | N/I | I/L |
| Mutation combination 40 | N/T | I/GG | V/L | N/L | Q/V | V/L |
| Mutation combination 41 | D/S | L/GG | V/V | Q/I | H/V | V/I |
| Mutation combination 42 | D/T | V/N | V/I | N/I | N/V | V/V |
| Mutation combination 43 | E/S | I/N | L/L | Q/V | Q/L | L/L |
| Mutation combination 44 | E/T | L/N | L/V | N/V | H/L | L/I |
| Mutation combination 45 | N/S | V/GG | L/I | Q/L | N/L | L/V |
| Mutation combination 46 | N/T | I/GG | I/L | N/L | N/V | I/L |
| Mutation combination 47 | D/S | L/GG | I/V | Q/I | N/I | I/I |
| Mutation combination 48 | D/T | V/N | I/I | N/I | N/L | I/V |
| Mutation combination 49 | E/S | I/N | V/L | Q/V | Q/V | V/L |
| Mutation combination 50 | E/T | L/N | V/V | N/V | Q/I | V/I |
| Mutation combination 51 | N/S | V/GG | V/I | Q/L | Q/L | V/V |
| Mutation combination 52 | N/T | I/GG | L/L | N/L | H/V | L/L |
| Mutation combination 53 | E/T | L/GG | L/V | Q/I | H/I | L/I |
| Mutation combination 54 | N/S | I/N | L/I | N/I | H/L | L/V |
| Mutation combination 55 | N/T | L/N | I/L | Q/V | N/V | I/V |
| Mutation combination 56 | D/S | V/GG | I/V | N/V | N/I | I/I |
| Mutation combination 57 | D/T | I/GG | I/I | Q/L | N/L | I/L |
| Mutation combination 58 | E/S | V/N | V/L | N/L | Q/V | V/V |
| Mutation combination 59 | E/T | I/N | V/V | Q/I | Q/I | V/I |
| Mutation combination 60 | N/S | L/N | V/I | N/I | Q/L | V/L |
| Mutation combination 61 | N/T | V/GG | L/L | Q/I | H/V | L/V |

3. The binding protein according to any one of claims 1-2, wherein the binding protein comprises at least 3 CDRs; alternatively, the binding protein comprises at least 6 CDRs;
further, the binding protein is one of a nanobody, F(ab')₂, Fab', Fab, Fv, scFv, a bispecific antibody and a minimal recognition unit of antibody.

4. The binding protein according to any one of claims 1-2, wherein the binding protein comprises light chain framework regions FR-L1, FR-L2, FR -L3 and FR-L4 with sequences correspondingly shown in SEQ ID NO: 1-4, and/or, heavy chain framework regions FR-H1, FR-H2, FR-H3 and FR-H4 with sequences correspondingly shown in SEQ ID NO: 5-8;
further, the binding protein further comprises a constant region sequence of antibody;
further, the constant region sequence is a sequence of any one constant region selected from IgG1, IgG2, IgG3, IgG4, IgA, IgM, IgE, and IgD;
further, the constant region is derived from species consisted of cattle, horse, dairy cow, pig, sheep, goat, rat, mouse, dog, cat, rabbit, camel, donkey, deer, mink, chicken, duck, goose, turkey, gamecock or human; further, the constant region is derived from the mouse;
a light chain constant region sequence is shown in SEQ ID NO: 9;
a heavy chain constant region sequence is shown in SEQ ID NO: 10.

5. An isolated nucleic acid, encoding the binding protein according to any one of claims 1-4.

6. A vector, comprising the nucleic acid according to claim 5.

7. A host cell, comprising the nucleic acid according to claim 5 or the vector according to claim 6.

8. A method for producing the binding protein according to any one of claims 1-4, the method comprising the steps of: culturing the host cell according to claim 7 in a culture medium, recovering a produced binding protein from the culture medium or from the cultured host cell.

9. Use of the binding protein according to any one of claims 1-4 in PCR.

10. A kit, comprising one or more of the binding proteins according to any one of claims 1-4, the isolated nucleic acid according to claim 5, or the vector according to claim 6.

11. A composition, comprising the binding protein according to any one of claims 1-4 and a Taq DNA polymerase.

12. The composition according to claim 11, wherein the composition further comprises 4 deoxynucleoside triphosphates.

13. The composition according to claim 11 or 12, wherein the composition further comprises a primer and/or a probe.

14. The composition according to any one of claims 11-13, wherein the composition further comprises MgCl₂.

15. The composition according to any one of claims 11-14, wherein the composition further comprises a nucleic acid as a template.

16. A method for amplifying a nucleic acid, comprising carrying out hot start PCR by using the binding protein according to any one of claims 1-4 or the composition according to any one of claims 11-15.

17. The method according to claim 16, wherein the hot start PCR is selected from a group consisted of multiplex PCR, real-time PCR, and real-time quantitative PCR.

18. Use of the binding protein according to any one of claims 1-4 in amplifying a nucleic acid.
